# EUROPÄISCHE PATENTSCHRIFT

(11) **EP 2 794 466 B1**
(45) Veröffentlichungstag und Bekanntmachung des Hinweises auf die Patenterteilung: **26.08.2015**
(21) Anmeldenummer: 12809617.9
(22) Anmeldetag: 20.12.2012
(51) Int. Cl.: C01B 31/02, C01B 31/18, C01B 3/24, C10J 3/00, C10K 3/06, C10G 2/00

(54) **VERFAHREN UND ANLAGE ZUR UMWANDLUNG VON KOHLENDIOXID IN KOHLENMONOXID**
PROCESS AND SYSTEM FOR CONVERSION OF CARBON DIOXIDE TO CARBON MONOXIDE
PROCÉDÉ ET INSTALLATION DE CONVERSION DE DIOXYDE DE CARBONE EN MONOXYDE DE CARBONE

(30) Priorität: 20.12.2011 DE 102011122562; 04.05.2012 DE 102012008933; 02.08.2012 DE 102012015314
(43) Veröffentlichungstag der Anmeldung: 29.10.2014
(73) Patentinhaber: CCP Technology GmbH, 81667 München (DE)
(72) Erfinder: KÜHL, Olaf, 17489 Greifswald (DE)
(74) Vertreter: Klang, Alexander H.
(86) Internationale Anmeldenummer: PCT/EP2012/005309
(87) Internationale Veröffentlichungsnummer: WO 2013/091878

(56) Entgegenhaltungen:
- EP-A2- 0 219 163
- GB-A- 873 213
- US-A- 4 353 713
- US-A- 5 164 054
- US-A1- 2003 024 806

## Beschreibung

Die vorliegende Erfindung bezieht sich auf ein Verfahren und eine Anlage zur Erzeugung von Kohlenmonoxid aus Kohlenwasserstoffen und CO₂.

Als Abgase im Rahmen der Energiegewinnung und im Zuge anderer industrieller Prozesse fallen große Mengen Kohlendioxid (CO₂) an, das als Klimaschadstoff angesehen wird. Es werden große Anstrengungen unternommen, die Entstehung von Kohlendioxid zu vermeiden. Weiterhin wird versucht, entstandenes Kohlendioxid aus den Rauchgasen abzutrennen und zu lagern. Ein Beispiel ist die CO₂-Speicherung oder Carbon-Capture-to-Storage, abgekürzt CCS, wobei das CO₂ aus Rauchgasen abgesondert, komprimiert und in geeigneten geologischen Formationen gelagert werden soll. Das CCS-Verfahren ist teuer, energieintensiv, begrenzt in den Lagerkapazitäten und wird von der betroffenen Bevölkerung aus verschiedenen Gründen stark abgelehnt. Die technische und politische Umsetzbarkeit scheint derzeit zumindest in Deutschland gescheitert.

Eine weitere Möglichkeit ist, Kohlendioxid als Rohstoff für andere industrielle Prozesse zu verwenden, beispielsweise als Rohstoff in der Kunststoffindustrie für Polyurethan, wie es von der Bayer AG im Project CO2RRECT verfolgt wird. Diese Verwendung als Rohstoff ist von der Menge her nur eine Nischenanwendung, da die Weltproduktion an den Zielprodukten der Anwendung viel zu gering ist, um einen nennenswerten Anteil des ausgestoßenen Kohlendioxides umzusetzen.

Keines dieser Konzepte hat bisher zu Anwendungen geführt, die größere Mengen Kohlendioxid binden oder in der Durchführung gesellschaftlich akzeptabel sind.

Synthesegas, oder abgekürzt Syngas, ist eine Gasmischung, aus Kohlenmonoxid und Wasserstoff, die außerdem Kohlendioxid aufweisen kann. Das Synthesegas wird beispielsweise durch Vergasung von Kohlenstoff enthaltendem Brennstoff zu einem gasförmigen Produkt, dem Synthesegas, erzeugt, welches einen gewissen Heizwert hat. Das Synthesegas hat etwa 50% der Energiedichte von natürlichem Erdgas. Das Synthesegas kann verbrannt und somit als Brennstoffquelle verwendet werden. Das Synthesegas kann weiterhin als Zwischenprodukt zur Erzeugung von anderen chemischen Erzeugnissen verwendet werden. Synthesegas kann beispielsweise durch Vergasung von Kohle oder Abfällen erzeugt werden. Bei der Erzeugung von Synthesegas wird beispielsweise Kohlenstoff mit Wasser oder ein Kohlenwasserstoff mit Sauerstoff zur Reaktion gebracht. Es gibt kommerziell verfügbare Technologien, um Synthesegas weiterzuverarbeiten, um schließlich Industriegase, Dünger, Chemikalien und andere chemische Produkte zu erzeugen. Bei den meisten bekannten Technologien (z.B. Water-Shift-Reaktion) zur Erzeugung und Umwandlung von Synthesegas besteht jedoch das Problem, dass bei der Synthese der benötigten Menge an Wasserstoff eine größere Menge überschüssiges CO₂ erzeugt wird, welches wiederum als klimaschädliches Gas in die Umwelt gelangt. Bei einer anderen bekannten Technik zur Herstellung von Synthesegas, der partiellen Oxidation von Methan nach der Gleichung 2 CH₄ + O₂ → 2 CO + 4 H₂, kann ein maximales Verhältnis von H₂:CO von 2,0 erreicht werden. Der Nachteil ist jedoch die Verwendung von reinem Sauerstoff, der energieaufwändig hergestellt werden muss.

Ferner wird auf die DD 276 098 A1 hingewiesen, die eine tiefere stoffliche Verwertung von Erdgas in Steamreforminganlagen beschreibt. Insbesondere ist unter anderem ein Verfahren zur Gewinnung von Ruß aus Erdgas mittels einer Bogenplasmapyrolyse beschrieben. Ferner zeigt die US 4 040 976 die Behandlung eines Kohlenstoff enthaltenden Materials, insbesondere Kohle mit Kohlendioxid zur Erzeugung eines Kohlenmonoxidgases. Dabei wird das Kohlendioxid zunächst mit Kohlenstoff enthaltendem Material vermischt und Anschließend in einem Rekator mit heißem Kohlendioxid rasch mit einer Rate von >500°C/s erhitzt und anschließend rasch abgekühlt, wobei die Heizphase zwischen 0.1 bis 50 ms liegt und die Gesamt-Verweilzeit der Reaktanden in einem Zeitraum von 10 ms bis 5s gehalten wird. Aus der US 4 190 636 ist darüber hinaus die Herstellung von Kohlenmonoxid in einem Plasma bekannt. Hierbei wird ein Plasma aus Kohlendioxid erzeugt, in das fester Kohlenstoff eingeleitet wird. Die sich ergebenden Produkte werden abgeschreckt und gefiltert, um Kohlenmonoxid zu erhalten.

EP 0 219 163 A2 offenbart ein Verfahren zum Erzeugen von Synthesegas, bei dem in einem ersten Reaktorraum Kohlenwasserstoffe zu Kohlenstoff und Wasserstoff aufgespaltet wird und der Kohlenstoff zur Reaktion mit Wasserdampf in einem zweiten Reaktionsraum überführt wird.

In GB 873 213 A ist ein Verfahren zum Erzeugen von Synthesegas beschrieben, bei dem zunächst an einem Katalysator Kohlenwasserstoffe zu Kohlenstoff aufgespaltet werden und dann der Katalysator mit dem daran abgelagerten Kohlenstoff mit Kohlenstoffdioxid beaufschlagt wird.

Es ist daher eine Aufgabe ein Verfahren zum Umwandeln von CO₂ vorzusehen, das in der Lage ist in effizienter Weise die von industriellen Prozessen ausgestoßene Kohlendioxidmenge zu verringern und gegebenenfalls die Erzeugung von benötigten chemischen Produkten ermöglicht.

Die Erfindung sieht ein Verfahren nach einem der Ansprüchen 1, 7 oder 9 sowie eine Vorrichtung nach einem der Ansprüche 18, 14 oder 16 vor. Weitere Ausgestaltungen ergeben sich aus den Unteransprüchen.

Insbesondere weist ein Verfahren zum Umwandeln von Kohlendioxid CO₂ in Kohlenmonoxid CO das Aufspalten eines Kohlenwasserstoff enthaltenden Fluids zu Kohlenstoff und Wasserstoff in einem Kohlenwasserstoffkonverten durch einen Energieeintrag, der wenigstens teilweise durch Wärme geleistet wird, auf, wobei der Kohlenstoff und der Wasserstoff nach der Aufspaltung eine Temperatur von wenigstens 200°C aufweist.

Wenigstens ein Teil des Kohlenstoffs, der aus der Aufspaltung gewonnen wurde, wird von dem Kohlenwasserstoffkonverter in einen CO₂-Konverter geleitet.

CO₂, das aus einem Verbrennungskraftwerk oder aus einem anderen Industrieprozess stammt, der geeignete Mengen an CO₂ erzeugt, wird in den CO₂-Konverter eingeleitet.

Das CO₂-Gas wird anschließend mit dem wenigstens einen Teil des Kohlenstoffes, der aus der Aufspaltung gewonnen wurde, gemischt, wobei sich der durch die Aufspaltung gewonnene Kohlenstoff beim Vermischen mit dem CO₂-Gas um höchstens 50% in °C hinsichtlich seiner Temperatur nach der Aufspaltung abgekühlt hat und wenigstens ein Teil des CO₂ -Gases und des durch die Aufspaltung gewonnenen Kohlenstoffes bei einer Temperatur von 800 bis 1700°C in CO umgewandelt wird. Dieses Verfahren ermöglicht auf einfache und effiziente Weise eine Umwandlung von CO₂ in CO, wobei wenigstens ein Teil einer für die Bereitstellung von Kohlenstoff (durch Aufspaltung eines Kohlenwasserstoffs) erforderlichen Energie in Form von Wärme für die Umwandlung eingesetzt wird.

Dies gilt insbesondere, wenn die Aufspaltung bei einer Temperatur über 1000°C erfolgt und der Kohlenstoff mit einer Temperatur von wenigstens 800°C mit dem CO₂-Gas vermischt wird, da in diesem Fall keine oder nur eine geringere zusätzliche Wärmemenge für die Umwandlung bereitgestellt werden muß. Bevorzugt wird die erforderliche Wärme zum Erreichen der Temperatur von 800 bis 1700°C (insbesondere ungefähr 1000°C) für die CO₂-Umwandlung im Wesentlichen vollständig durch die Wärme, die für die Aufspaltung des Kohlenwasserstoff enthaltenden Fluids eingesetzt wird, bereitgestellt. Im Wesentlichen bedeutet hier, dass wenigstens 80%, insbesondere wenigstens 90% der erforderlichen Wärme aus dem Aufspaltungsprozeß stammen.

Bei einer Ausführungsform werden der durch die Aufspaltung gewonnene Kohlenstoff und der durch die Aufspaltung gewonnene Wasserstoff gemeinsam mit dem CO₂-Gas vermischt. Wasserstoff beinträchtigt die Umwandlung nicht und kann als zusätzlicher Wärmeträger dienen, was besonders vorteilhaft ist, wenn der Kohlenstoff und der Wasserstoff eine Temperatur von 1000°C (einer bevorzugten Umwandlungstemperatur) oder höher besitzen. In diesem Fall liegt nach der Umwandlung kein reines CO sondern ein Synthesegas vor.

Alternativ kann der durch die Aufspaltung gewonnene Kohlenstoff vor dem Vermischen mit dem CO₂-Gas von dem durch die Aufspaltung gewonnenen Wasserstoff getrennt werden.

Zum Erhöhen der Energieeffizienz des Verfahrens kann wenigstens ein Teil der Wärme wenigstens eines Teils des durch die Aufspaltung gewonnenen Kohlenstoffs und/oder Wasserstoffes zum Erwärmen des CO₂-Gases vor dem Vermischen mit dem Kohlenstoff und/oder zum Erwärmen eines Prozessraums, in dem das CO₂-Gases mit dem Kohlenstoff vermischt wird, verwendet werden. In diesem Sinne sei auch bemerkt, dass das CO nach der Umwandlung eine Temperatur von 800 bis 1700°C aufweist und wenigstens ein Teil seiner Wärme zum Vorwärmen des CO₂-Gases vor dem Vermischen mit dem Kohlenstoff verwendet werden kann. Auch ist es möglich, dass wenigstens ein Teil der Wärme wenigstens eines Teils des durch die Aufspaltung gewonnenen Kohlenstoffs und/oder Wasserstoffes und/oder des CO nach der Umwandlung zur Erzeugung von elektrischem Strom verwendet wird, der insbesondere als Energieträger für den Energieeintrag für das Aufspalten des Kohlenwasserstoff enthaltenden Fluids bereitgestellt werden kann.

Bevorzugt erfolgt der Energieeintrag in den Kohlenwasserstoff zu seiner Aufspaltung primär über ein Plasma. Dies ist eine besonders direkte und somit effiziente Form des Energieeintrags. Bevorzugt wird die Aufspaltung in einem Kvaerner-Reaktor durchgeführt, der eine kontinuierliche Aufspaltung eines Stromes an Kohlenwasserstoffen ermöglicht.

Bei dem Verfahren zum Erzeugen eines Synthesegases wird zunächst CO₂ in CO wie oben beschrieben umgewandelt und dem CO anschließend Wasserstoff beigemischt. Bevorzugt stammt der Wasserstoff aus einer Aufspaltung eines Kohlenwasserstoff enthaltenden Fluids zu Kohlenstoff und Wasserstoff durch einen Energieeintrag, der wenigstens teilweise durch Wärme geleistet wird. Die Aufspaltung kann somit einerseits den erforderlichen Kohlenstoff zur CO₂-Umwandlung und anderseits den erforderlichen Wasserstoff in einem Arbeitsgang zur Verfügung stellen. Bei einer Ausführungsform wird wenigstens ein Teil des Wasserstoffs durch Aufspalten eines Kohlenwasserstoff enthaltenden Fluids bei einer Temperatur unter 1000°C, insbesondere unter 600°C mittels eines Mikrowellenplasmas erzeugt. Dort wo zusätzlicher Wasserstoff (mehr als bei der Herstellung des für die CO₂-Umwandlung erforderlichen Kohlenstoffs anfällt) für die Einstellung eines Mischungsverhältnisses eines Synthesegases benötigt wird, kann dieser bevorzugt energieeffizient bei niedrigeren Temperaturen aus einem Kohlenwasserstoff enthaltenden Fluid abgespalten werden. Bevorzugt wird das Verhältnis CO zu Wasserstoff im Synthesegas auf einen Wert von 1:1 bis 1:3, insbesondere auf einen Wert von ungefähr 1:2,1 eingestellt.

Bei dem Verfahren zum Erzeugen von synthetischen, funktionalisierten und/oder nicht-funktionalisierten Kohlenwasserstoffen, wird zunächst ein Synthesegas wie oben beschrieben erzeugt und dieses mit einem geeigneten Katalysator in Kontakt gebracht, um eine Umwandlung des Synthesegases in synthetische, funktionalisierte und/oder nicht-funktionalisierte Kohlenwasserstoffe zu bewirken, wobei die Temperatur des Katalysators und oder des Synthesegases auf einen vorbestimmten Temperaturbereich gesteuert oder geregelt wird. Dabei kann das Synthesegas vorab oder auch direkt im Katalysator durch Mischen von CO mit Wasserstoff erzeugt werden.

Bei einer Ausführungsform wird die Umwandlung des Synthesegases mittels eines Fischer-Tropsch-Verfahrens, insbesondere mittels eines SMDS-Verfahrens bewirkt. Alternativ wird die Umwandlung des Synthesegases mittels eines Bergius-Pier-Verfahrens, eines Pier-Verfahrens oder einer Kombination aus einem Pier-Verfahren mit einem MtL-Verfahren bewirkt. Die Wahl des Verfahrens bestimmt im Wesentlichen die Form der synthetischen, funktionalisierten und/oder nicht-funktionalisierten Kohlenwasserstoffe.

Bevorzugt ist das aufzuspaltende Kohlenwasserstoff enthaltende Fluid Erdgas, Methan, Flüssiggas, Schweröl oder eine Mischung derselben.

Die Vorrichtung zum Umwandeln von Kohlendioxid CO₂ in Kohlenmonoxid CO besitzt einen Kohlenwasserstoffkonverter zum Aufspalten eines Kohlenwasserstoff enthaltenden Fluids in Kohlenstoff und Wasserstoff, der wenigstens einen Prozessraum mit wenigstens einem Eingang für ein Kohlenwasserstoff enthaltendes Fluid und wenigstens einem Ausgang für Kohlenstoff und/oder Wasserstoff und wenigstens eine Einheit zum Einbringen von Energie in den Prozessraum, die wenigstens teilweise aus Wärme besteht, aufweist. Ferner weist die Vorrichtung einen CO₂-Konverter zur Umwandlung von CO₂ in CO auf, der wenigstens einen weiteren Prozessraum mit wenigstens einem Eingang für CO₂ zum Einleiten von CO₂ aus einem Verbrennungskraftwerk oder einem anderen Industrieprozess in den CO₂-Konverter, wenigstens einem Eingang für wenigstens Kohlenstoff und wenigstens einem Ausgang aufweist, wobei der Eingang für wenigstens Kohlenstoff direkt mit dem wenigstens einem Ausgang des Kohlenwasserstoffkonverters verbunden ist. Dabei soll direkt hier umschreiben, dass im Betrieb aus dem Kohlenwasserstoffkonverter austretender Kohlenstoff auf seinem Weg zum CO₂-Konverter um nicht mehr als 50% seiner Temperatur in °C, bevorzugt um nicht mehr als 20% abkühlt, ohne dass zusätzliche Energie zum Wärmen des Kohlenstoffs eingesetzt wird. Zwischen dem Ort der Aufspaltung und dem wenigstens einen Ausgang des Kohlenwasserstoffkonverters kann eine Trenneinheit vorgesehen sein, welche den Kohlenstoff vom Wasserstoff trennt. Diese kann einen Teil des Kohlenwasserstoffkonverters bilden oder auch außerhalb des Kohlenwasserstoffkonverters als separate Einheit vorgesehen sein. Eine Trenneinheit zwischen dem Ausgang des Kohlenwasserstoffkonverters und dem Eingang eines CO₂-Konverters behindert eine direkte Verbindung nicht, solange die obige Bedingung beibehalten wird.

Bevorzugt ist die wenigstens eine Einheit zum Einbringen von Energie in den Prozessraum so ausgebildet, dass sie wenigstens lokal Temperaturen über 1000°C, insbesondere über 1500°C erzeugen kann. Bei einer Ausführungsform ist die wenigstens eine Einheit zum Einbringen von Energie in den Prozessraum eine Plasmaeinheit. Insbesondere wenn die Temperatur für die Aufspaltung unter 1000°C gehalten werden soll, weist die wenigstens eine Einheit zum Einbringen von Energie in den Prozessraum bevorzugt eine Mikrowellen-Plasmaeinheit auf.

Für eine besonders einfache Ausgestaltung der Vorrichtung wird der Prozessraum des CO₂-Konverters durch ein Auslassrohr des Kohlenwasserstoffkonverters gebildet, das mit einer Einspeisung für CO₂-Gas verbunden ist.

Bei einer Ausführungsform der Erfindung sind im Bereich des Kohlenwasserstoffkonverters eine Trenneinheit zum Trennen des durch die Aufspaltung entstandenen Kohlenstoffs und des Wasserstoffs und getrennte Ausgänge für die getrennten Stoffe aus der Trenneinheit vorgesehen, wobei der Ausgang für Kohlenstoff mit dem CO₂-Konverter verbunden ist.

Bei dem Kohlenwasserstoffkonverter handelt es sich bevorzugt um einen Kvaerner-Reaktor, der die erforderlichen Temperaturen im Dauerbetrieb für eine kontinuierliche Aufspaltung eines Kohlenwasserstoff enthaltenden Fluids vorsehen kann.

Die Vorrichtung zum Erzeugen eines Synthesegases, weist eine Vorrichtung des zuvor beschriebenen Typs sowie wenigstens eine separate Zuleitung für Wasserstoff in den CO₂-Konverter oder eine stromabwärts befindlichen Mischraum auf. Eine solche Vorrichtung ermöglicht eine einfache und effiziente Erzeugung eines Synthesegases aus CO₂ und einem Kohlenwasserstoff enthaltenden Fluid.

Bei einer Ausführungsform weist die Vorrichtung zum Erzeugen eines Synthesegases wenigstens einem weiteren Kohlenwasserstoffkonverter zum Aufspalten eines Kohlenwasserstoff enthaltenden Fluids in Kohlenstoff und Wasserstoff auf, Der wenigstens eine weitere Kohlenwasserstoffkonverter weist wieder wenigstens einen Prozessraum mit wenigstens einem Eingang für das Kohlenwasserstoff enthaltende Fluid, wenigstens eine Einheit zum Einbringen von Energie in den Prozessraum, die wenigstens teilweise aus Wärme besteht, und eine Trenneinheit zum Trennen des durch die Aufspaltung entstandenen Kohlenstoffs und des Wasserstoffs mit getrennten Ausgängen für Kohlenstoff und Wasserstoff auf, wobei der Ausgang für Wasserstoff mit der separaten Zuleitung für Wasserstoff verbunden ist. Dabei ist der wenigstens eine weitere Kohlenwasserstoffkonverter aus Gründen der Energieeffizienz bevorzugt des Typs, der ein Aufspaltung bei Temperaturen unter 1000°C, insbesondere unter 600°C mittels eines Mikrowellenplasmas bewirkt.

Bei der Vorrichtung zum Umwandeln eines Synthesegases in synthetische, funktionalisierte und/oder nicht-funktionalisierte Kohlenwasserstoffe, ist eine Vorrichtung zum Erzeugen eines Synthesegases des obigen Typs und ein CO-Konverter vorgesehen. Der CO-Konverter weist einen Prozessraum, in dem ein Katalysator angeordnet ist, Mittel zum Leiten des Synthesegases in Kontakt mit dem Katalysator, und eine Steuereinheit zum Steuern oder Regeln der Temperatur des Katalysators und/oder des Synthesegases auf eine vorbestimmte Temperatur auf. Dabei können Teile der Vorrichtung zum Erzeugen eines Synthesegases im CO-Konverter integriert sein, wie beispielsweise ein Mischraum für CO und zusätzlichen Wasserstoff. Bei einer Ausführungsform weist der CO-Konverter einen Fischer-Tropsch-Konverter, insbesondere einen SMDS-Konverter auf. Alternativ kann der CO-Konverter auch ein Bergius-Pier-Konverter, einen Pier-Konverter oder eine Kombination eines Pier-Konverters mit einen MtL-Konverter aufweisen. Auch ist es möglich, dass mehrere CO-Konverter desselben oder auch unterschiedlicher Typen in der Vorrichtung vorhanden sind.

Bevorzugt weist die Vorrichtung eine Steuereinheit zum Steuern oder Regeln des Drucks des Synthesegases im CO-Konverter auf.

Die Erfindung wird nachfolgend unter Bezugnahme auf bestimmte Ausführungsformen anhand der Zeichnungen näher erläutert; in den Zeichnungen zeigt:
- Fig. 1: eine schematische Darstellung einer Anlage zur Erzeugung von Kohlenmonoxid;
- Fig. 2: eine schematische Darstellung einer Anlage zur Erzeugung von Synthesegas;
- Fig. 3: eine schematische Darstellung einer Anlage zur Erzeugung von funktionalisiertem und/oder nicht-funktionalisiertem Kohlenwasserstoff;
- Fig. 4: eine schematische Darstellung einer weiteren Anlage zur Erzeugung von funktionalisiertem und/oder nicht-funktionalisiertem Kohlenwasserstoff gemäß einer weiteren Ausführungsform;
- Fig. 5: eine schematische Darstellung einer Anlage zur Erzeugung von funktionalisiertem und/oder nicht-funktionalisiertem Kohlenwasserstoff gemäß einer weiteren Ausführungsform;
- Fig. 6: eine schematische Darstellung einer Anlage zur Erzeugung von funktionalisiertem und/oder nicht-funktionalisiertem Kohlenwasserstoff gemäß einer weiteren Ausführungsform;
- Fig. 7: eine schematische Darstellung einer Anlage zur Erzeugung von Synthesegas gemäß einer weiteren Ausführungsform; und
- Fig. 8: eine schematische Darstellung einer Anlage zur Erzeugung von funktionalisiertem und/oder nicht-funktionalisiertem Kohlenwasserstoff gemäß einer weiteren Ausführungsform.

Es sei bemerkt, dass sich in der folgenden Beschreibung die Ausdrücke oben, unten, rechts und links sowie ähnliche Angaben auf die in den Figuren dargestellten Ausrichtungen bzw. Anordnungen beziehen und nur zur Beschreibung der Ausführungsbeispiele dienen. Diese Ausdrücke sind jedoch nicht im einschränkenden Sinne zu verstehen. Ferner werden in den unterschiedlichen Figuren zum Teil dieselben Bezugszeichen verwendet, sofern gleiche oder ähnliche Teile bezeichnet werden.

In der folgenden Beschreibung werden Prozesse und Vorrichtungen beschrieben, die "heiße" Stoffe oder "heiße" Prozesse ausführen. Im Zusammenhang mit dieser Beschreibung soll der Ausdruck "heiß" eine Temperaturüber 200°C und vorzugsweise über 300°C beschreiben.

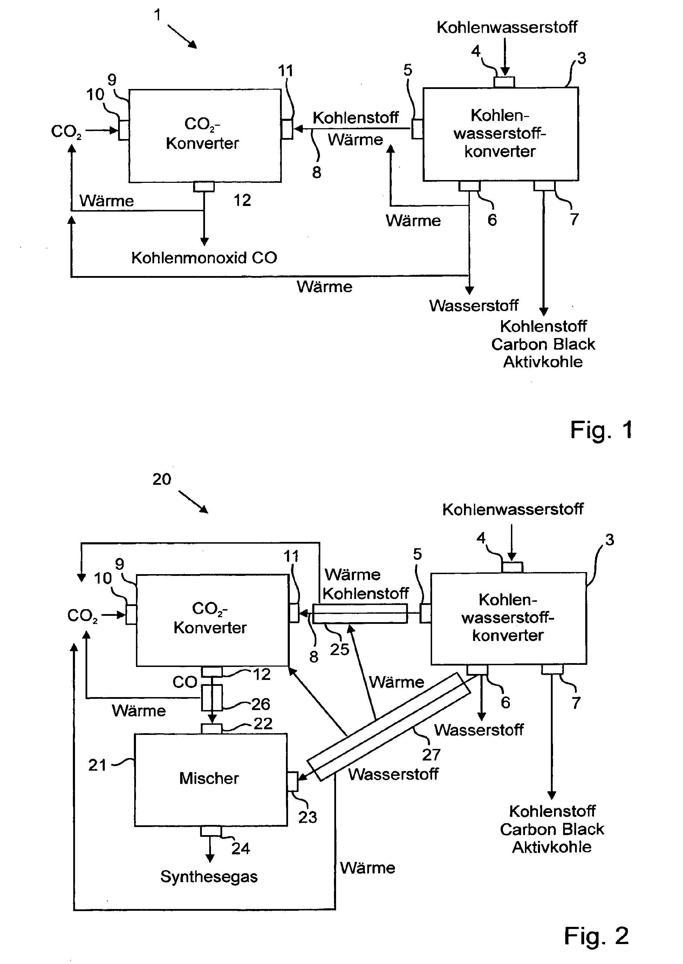

Fig. 1 stellt schematisch eine Anlage 1 zur Umwandlung von Kohlendioxid in Kohlenmonoxid dar. Aus der Fig. 1 wird auch der grundlegende Verfahrensablauf zur Umwandlung von Kohlendioxid in Kohlenmonoxid gemäß dieser Beschreibung deutlich.

Die Anlage 1 weist einen Kohlenwasserstoffkonverter 3 auf, welcher einen Kohlenwasserstoffeingang 4 sowie einen ersten Kohlenstoffausgang 5, einen optionalen Wasserstoffausgang 6 sowie einen optionalen zweiten Kohlenstoffausgang 7 aufweist. Die Anlage 1 zur Erzeugung von Kohlenmonoxid weist weiter einen CO₂-Konverter 9 mit einem CO₂-Eingang 10 einem Kohlenstoffeingang 11 (auch als C-Eingang bezeichnet) und einen Ausgang 12 auf. Der Kohlenwasserstoffkonverter 3 und der CO₂-Konverter 9 sind derart angeordnet, dass der Kohlenstoffausgang 5 des Kohlenwasserstoffkonverters 3 über eine direkte Verbindung 8 mit dem Kohlenstoffeingang 11 des CO₂-Konverters 9 verbunden ist, wobei der Ausgang 5 auch direkt den Kohlenstoffeingang11 des CO₂-Konverters 9 bilden kann. So kann Kohlenstoff aus dem Kohlenwasserstoffkonverter 3 direkt in den CO₂-Konverter 9 transportiert werden.

Der Kohlenwasserstoffkonverter 3 ist irgendein Kohlenwasserstoffkonverter, der eingespeiste Kohlenwasserstoffe in Kohlenstoff und Wasserstoff umwandeln bzw. aufspalten kann. Der Kohlenwasserstoffkonverter 3 weist einen Prozessraum mit einem Einlaß für ein Kohlenwasserstoff enthaltendes Fluid, wenigstens eine Einheit zum Einbringen von Aufspaltungsenergie in das Fluid und wenigstens einen Auslaß auf. Die Aufspaltungsenergie wird wenigstens teilweise durch Wärme zur Verfügung gestellt, die beispielsweise durch ein Plasma erzeugt wird. Sie kann aber auch auf andere Weise zur Verfügung gestellt werden und wenn primär eine Aufspaltung über Wärme erfolgt, so sollte das Fluid auf über 1000°C insbesondere auf eine Temperatur über 1500°C aufgeheizt werden.

Bei der dargestellten Ausführungsform wird ein Kvaerner Reaktor eingesetzt, der mittels eines Plasmabogens in einem Plasma-Brenner die erforderliche Wärme zur Verfügung stellt. Es sind aber auch andere Reaktoren bekannt, die bei niedrigeren Temperaturen insbesondere unter 1000°C arbeiten und neben der Wärme zusätzliche Energie in den Kohlenwasserstoff einbringen, wie beispielsweise über ein Mikrowellenplasma. Wie Nachfolgend noch näher erläutert wird, zieht die Erfindung beide Reaktortypen (und auch solche die ohne ein Plasma arbeiten) in betracht, insbesondere auch in Kombination miteinander. Kohlenwasserstoffkonverter die bei einer Temperatur von mehr als 1000°C arbeiten werden nachfolgend als Hochtemperatur-Reaktoren bezeichnet, während solche, die bei Temperaturen unter 1000°C arbeiten, insbesondere bei einer Temperatur zwischen 200°C und 1000°C, als Niedertemperatur-Reaktoren bezeichnet werden.

In dem Reaktor werden mittels Wärme und/oder einem Plasma Wasserstoff und Kohlenstoff aus Kohlenwasserstoffen (CnHm) generiert. Die Kohlenwasserstoffe werden dabei bevorzugt in Gasform in den Reaktor eingebracht. Bei unter Normalbedingungen flüssigen Kohlenwasserstoffen können diese vor dem Einbringen in den Reaktor in Gasform gebracht werden, oder sie könnten auch in einer fein zerstäubten Form eingeleitet werden. Beide Formen werden nachfolgend als Fluide bezeichnet.

Die Auftrennung der Kohlenwasserstoffe sollte möglichst unter Ausschluß von Sauerstoff erfolgen, um die unerwünschte Bildung von Kohlenstoffoxiden oder Wasser zu unterbinden. Geringe Mengen an Sauerstoff, die beispielsweise mit dem Kohlenwasserstoffen eingebracht werden, sind aber auch wiederum für den Prozeß nicht schädlich.

Bei dem oben beschriebenen Kvaerner Reaktor werden Kohlenwasserstoff enthaltende Fluide in einem Plasmabrenner bei hoher Temperatur in reinen Kohlenstoff (beispielsweise in Form von Aktivkohle, Carbon Black, Graphit oder Industrieruß) und Wasserstoff und ggf. Verunreinigungen getrennt. Die Kohlenwasserstoffe enthaltenden Fluide als Eingangsstoffe für den Kohlenwasserstoffkonverter 3 sind beispielsweise Methan, Erdgas, Biogase, Flüssiggase oder Schweröl, es können aber auch synthetische, funktionalisierte und/oder nicht-funktionalisierte Kohlenwasserstoffe als Eingangsstoffe für den Kohlenwasserstoffkonverter 3 verwendet werden. Nach der ursprünglichen Trennung liegen die Elemente in der Regel als eine Mischung, insbesondere in Form eines Aerosols vor. Diese Mischung kann, wie nachfolgend beschrieben wird, in dieser Form einem weiteren Prozeß zugeführt werden, oder sie kann auch in einer entsprechenden, nicht dargestellten Trenneinheit in ihre Einzelelemente getrennt werden. Eine solche Trenneinheit wird im Rahmen dieser Anmeldung als Teil des Kohlenwasserstoffkonverters 3 angesehen, auch wenn sie natürlich auch als getrennte Einheit ausgeführt sein kann. Wenn keine Trenneinheit vorgesehen ist, dann ist der Kohlenstoffausgang 5 der einzige Ausgang des Kohlenwasserstoffkonverters 3 sein, der eine Mischung (ein Aerosol) aus Kohlenstoff und Wasserstoff direkt in den CO₂-Konverter 9 leitet. Mit einer Trenneinheit kann, über den Kohlenstoffausgang 5 wenigstens teilweise vom Wasserstoff getrennter Kohlenstoff in den CO₂-Konverter 9 geleitet werden. Über die optionalen Ausgänge 6 und 7 können dann abgetrennter Wasserstoff und ggf. weiterer Kohlenstoff ausgeleitet werden.

Der CO₂-Konverter 9 kann irgendein geeigneter CO₂-Konverter sein, der Kohlenmonoxid (CO) aus Kohlenstoff (C) und Kohlendioxid (CO₂) erzeugen kann. In der Ausführungsform der Fig. 1 arbeitet der CO₂-Konverter 9 nach einem Teil der in der Technik bekannten Hochofenreaktion, welche bei Temperaturen zwischen ca. 750°C und 1200°C ohne die Notwendigkeit eines Katalysators abläuft. Vorzugsweise arbeitet der CO₂-Konverter 9 bei einer Temperatur zwischen 800°C und 1000°C, wobei die für das Erreichen dieser Temperatur erforderliche Wärme primär durch das Ausgangsmaterial des Kohlenwasserstoffkonverters 3 zur Verfügung gestellt wird, wie nachfolgend noch näher erläutert wird.. Im CO₂-Konverter 9 wird CO₂ über heißen Kohlenstoff geleitet oder mit diesem (und ggf. Wasserstoff) vermischt, um gemäß der chemischen Gleichung CO₂ + C → 2 CO umgewandelt zu werden. Der CO₂-Konverter 9 arbeitet am besten bei dem Boudouard-Gleichgewicht und einer Temperatur von 1000°C. Bei Temperaturen von 800°C werden etwa 94% Kohlenmonoxid geliefert, und bei Temperaturen um 1000°C werden etwa 99% Kohlenmonoxid geliefert. Ein weiterer Temperaturanstieg bringt keine wesentlichen Änderungen mehr mit sich.

Der Betrieb der Anlage 1 zur Umwandlung von Kohlendioxid in Kohlenmonoxid wird nachfolgend unter Bezugnahme auf die Fig. 1 näher erläutert. Dabei wird im Nachfolgenden davon ausgegangen, dass der Kohlenwasserstoffkonverter 3 ein Hochtemperatur Reaktor des Kvaerner Typs ist. Kohlenwasserstoffe enthaltende Fluide (insbesondere in Gasform) werden über den Kohlenwasserstoffeingang 4 in den Kohlenwasserstoffkonverter 3 eingeleitet. Wenn der Kohlenwasserstoff beispielsweise Methan (CH4) ist, so entstehen 1 mol Kohlenstoff und 2 mol Wasserstoff aus 1 mol Methan. Die Kohlenwasserstoffe werden in dem Kohlenwasserstoffkonverter 3 bei etwa 1600°C gemäß der folgenden Reaktionsgleichung umgewandelt, wobei die zugeführte Energie Wärme ist, die im Plasma mittels elektrischer Energie erzeugt wird:

CₙHₘ + Energie → n C + m/2 H₂

Bei entsprechender Prozeßführung ist der Kvaerner Reaktor in der Lage im kontinuierlichen Betrieb eine nahezu 100% Umwandlung des Kohlenwasserstoffs in seine Bestandteile zu erreichen.

Im Nachfolgenden wird davon ausgegangen, dass der Kohlenstoff und der Wasserstoff im Kohlenwasserstoffkonverter 3 getrennt und weitestgehend getrennt ausgeleitet werden. Es ist aber auch möglich, dass eine Trennung nicht erfolgt, und der Kohlenstoff und der Wasserstoff als Gemisch ausgeleitet und dem CO₂-Konverter 9 zugeführt werden. Der Wasserstoff beeinträchtigt nicht den Umwandlungsprozeß im CO₂-Konverter 9, kann aber als zusätzlicher Wärmeträger dienen. Der Kohlenstoff wird zumindest teilweise direkt über den Kohlenstoffausgang 5 in den Kohlenstoffeingang 11 des CO₂-Konverters 9 geleitet. Dabei soll das "direkte" leiten vom Ausgang 5 des Kohlenwasserstoffkonverters 3 zum Kohlenstoffeingang 11 des CO₂-Konverters 9 alle solche Varianten umfassen, bei denen keine Abkühlung von mehr als 50% bezogen auf die Temperatur (vorzugsweise von nicht mehr als 80%) der geleiteten Stoffe auftritt. Da der aus dem Kohlenwasserstoffkonverter 3 austretende Kohlenstoff eine hohe Temperatur bevorzugt über 1000°C aufweist, kann die darin enthaltene Wärmeenergie zum Erhalten der erforderlichen Temperatur für den Umwandlungsprozesses im CO₂-Konverter 9 verwendet werden, der vorzugsweise bei einer Temperatur von ca. 1000°C arbeitet.

Die Verbindung 8 zwischen dem Kohlenwasserstoffkonverter 3 und dem CO₂-Konverter 9 ist so ausgestaltet, dass sich der Kohlenstoff auf dem Weg vom Kohlenwasserstoffkonverter 3 zum CO₂-Konverter 9 nicht stark abkühlt (um weniger als 50%, bevorzugt weniger als 20% bezogen auf die Temperatur). Beispielsweise kann die Verbindung 8 besonders isoliert und/oder sogar aktiv beheizt sein, wobei dem System bevorzugt - neben der Wärmezufuhr im Kohlenwasserstoffkonverter 3 - keine weitere Wärme zugeführt wird. Der im Kohlenwasserstoffkonverter 3 erzeugte Wasserstoff enthält aufgrund der Betriebstemperatur im Kohlenwasserstoffkonverter 3 ebenfalls Wärmeenergie. Daher besteht eine Möglichkeit des Beheizens der Verbindung 8 darin, die Wärmeenergie des aus dem Wasserstoffausgang 6 herausgeleiteten Wasserstoffes, direkt oder indirekt über eine Wärmetauscheranordnung zum Beheizen der Verbindung 8 zwischen Kohlenwasserstoffkonverter 3 und CO₂-Konverter 9 zu verwenden.

Im CO₂-Konverter 9 wird CO₂, das über den CO₂-Eingang 10 des CO₂-Konverters 9 eingeleitet wird, über den heißen Kohlenstoff geleitet und/oder mit diesem vermischt. Der CO₂-Konverter 9 arbeitet am besten beim Boudouard-Gleichgewicht, welches sich bei der Umsetzung von Kohlendioxid mit heißem Kohlenstoff einstellt. Die Reaktion, welche dem Fachmann bekannt ist, ist abhängig von Druck und Temperatur und wird hier nicht im Einzelnen beschrieben. Entweder die Menge des in den CO₂-Konverter 9 eingeleiteten CO₂ oder die Menge des Kohlenstoffs kann über geeignete Mittel gesteuert und/oder geregelt werden.

CO₂ + C → 2CO; ΔH = +172,45 kJ/mol

Das CO₂ stammt aus einem Verbrennungskraftwerk (Kohle-, Gas- und/oder Ölkraftwerk) oder aus einem anderen Industrieprozeß (wie Beispielsweise der Stahl- oder Zementherstellung), der geeignete Mengen an CO₂ erzeugt. Abhängig von der Temperatur des CO₂ aus der CO₂-Quelle, ist es vorteilhaft, das in den CO₂-Eingang 10 des CO₂-Konverters 9 eingeleitete CO₂ vorzuwärmen, da der CO₂-Konverter 9 bei einer Temperatur zwischen 800 und 1200°C arbeitet. Eine Vorwärmung des CO₂ kann beispielsweise erreicht werden, indem die in dem heißen Wasserstoff enthaltene Wärmeenergie direkt oder indirekt über eine Wärmetauscheranordnung zum Vorwärmen des CO₂ verwendet wird. Bevorzugt reicht aber auch die im Kohlenstoff enthaltene Wärme, um das CO₂ auf die gewünschte Temperatur zu bringen. Nur für den Fall, dass die im Kohlenwasserstoffkonverter 3 erzeugte Wärme nicht ausreicht, um die gewünschte Umwandlungstemperatur von ungefähr 1000°C zu erreichen, kann eine optionale zusätzliche Heizeinheit zum Erwärmen des CO₂-Konverters 9 oder der darin befindlichen Elemente vorgesehen sein. Eine solche kann auch als eine Vorheizeinheit im Bereich einer Zuleitung für das CO₂ oder den Kohlenstoff eingesetzt werden. Sie kann auch nur für den Start der Anlage eingesetzt werden, um zunächst den CO₂-Konverter 9 oder medienführende Teile der Anlage auf eine Anfangstemperatur zu bringen, damit das System schneller einen gewünschten Temperaturzustand erreicht. Die Aufheizung aller medienführenden Teile rein über die im Kohlenwasserstoffkonverter 3 erzeugte Wärme könnte anfangs zu lange dauern.

Aus dem CO₂-Konverter 9 tritt heißes Kohlenmonoxid (CO) bei einer Temperatur von ungefähr 800 bis 1000°C aus (abhängig von der Betriebstemperatur des CO₂-Konverters 9). Das aus dem CO₂-Konverter 9 austretende Kohlenmonoxid enthält also ebenfalls Wärmeenergie, die direkt oder indirekt über einen in Fig. 1 nicht gezeigten Wärmetauscher beispielsweise zum Vorwärmen des in den CO₂-Eingang 10 eingeleiteten CO₂ verwendet werden kann.

Wie oben erwähnt, kann der Kohlenwasserstoffkonverter 3 einen zweiten Kohlenstoffausgang 7 zum Ausleiten von Kohlenstoff aufweisen. Der im Kohlenwasserstoffkonverter 3 erzeugte Kohlenstoff kann - nach einer entsprechenden Trennung (oder auch als C-H₂ Mischung) in unterschiedlichen Anteilen aus dem ersten Kohlenstoffausgang 5 und dem zweiten Kohlenstoffausgang 7 herausgeleitet werden. Der zweite Kohlenstoffausgang 7 wird verwendet, um gegebenenfalls einen Anteil des erzeugten Kohlenstoffes zu entnehmen, der nicht im CO₂-Konverter 9 zur Erzeugung von Kohlenmonoxid verwendet wird. Der aus dem zweiten Kohlenstoffausgang 7 entnommene Kohlenstoff kann als Aktivkohle, Graphit, Carbon Black oder andere Modifikation, wie Carbon Cones oder Carbon Discs, entnommen werden. Je nach Form und Qualität des entnommenen Kohlenstoffes kann der entnommene Kohlenstoff als Rohstoff in der chemischen Industrie oder für die Elektronikindustrie verwendet werden. Mögliche Anwendungen sind beispielsweise die Halbleiterherstellung, Reifenherstellung, Tinten, Toner oder ähnliche Produkte. Der vom Kohlenwasserstoffkonverter 3 erzeugte Kohlenstoff ist ein hochreiner Rohstoff, der gut weiterverarbeitet werden kann.

Mit Hilfe des oben dargestellten Verfahrens zur Umwandlung von Kohlendioxid in CO ist es möglich, den heißen Kohlenstoff aus dem Kohlenwasserstoffkonverter 3 mit warmem bis heißem Kohlendioxid aus der Abluft von Industrieprozessen im CO₂-Konverter 9 ohne oder wenigstens ohne nennenswerte externe Energiezufuhr zu Kohlenmonoxid umzusetzen. Bevorzugt sollten wenigstens 80%, insbesondere wenigstens 90% der zum Erreichen der Umwandlungstemperatur erforderlichen Wärme aus dem Kohlenwasserstoffkonverter 3 stammen.

In Fig. 2 ist eine Anlage 20 zur Erzeugung von Synthesegas gezeigt, welche die oben beschriebenen Elemente der Anlage 1 zur Erzeugung von Kohlenmonoxid sowie einen Mischer 21 aufweist, der einen CO-Eingang 22 zum Einleiten von Kohlenmonoxid und einen H₂-Eingang 23 zum Einleiten von Wasserstoff sowie einen Synthesegasausgang 24 zum Ausleiten von Synthesegas aufweist. Der CO-Eingang 22 ist mit dem CO-Ausgang 12 des CO₂-Konverters 9 verbunden. Der H₂-Eingang 23 des Mischers 21 ist mit dem H₂-Ausgang 6 des Kohlenwasserstoffkonverters 3 verbunden. Wie der Fachmann erkennen kann, wird bei der Ausführung, bei der eine C-H₂ Mischung über den Kohlenstoffausgang 5 in den CO₂-Konverter 9 geleitet wird, automatisch ein Synthesegas mit einem Mischungsverhältnis von CO-H₂ von ungefähr 1:1 erzeugt. In diesem Fall kann ggf. ein Mischer 21 entfallen oder zur Einstellung eines anderen Mischungsverhältnisses eingesetzt werden.

Der Mischer 21 kann irgendeine geeignete Vorrichtung zum Vermischen von Gasen sein und zum Beispiel in einem einfachen Fall die Form einer Rohrleitung mit entsprechenden Einlässen und einem Auslaß einnehmen. Mit Hilfe des Mischers 21 und insbesondere über eine Steuerung/Regelung des (zusätzlich) über den H₂-Eingang 23 des Mischers 21 eingeleiteten Wasserstoff kann die Mischung des Synthesegases am Synthesegasausgang 24 so beeinflusst werden, dass eine für nachfolgende Prozesse erforderliche Zusammensetzung erreicht wird.

Für viele Prozesse, beispielsweise die Fischer-Tropsch-Synthese, soll das Verhältnis von Wasserstoff zu CO möglichst hoch sein. Mit Hilfe des Mischers 21 kann ein beliebiges Verhältnis von Wasserstoff zu CO am Synthesegasausgang 24 eingestellt werden. Es wird in Betracht gezogen, dass nur ein Teil des CO und/oder nur ein Teil des Wasserstoffes in den Mischer 21 eingeleitet wird, während die nicht in den Mischer eingeleiteten Teile von CO und Wasserstoff jeweils als reines Gas aus dem Prozess entnommen werden können. Daher ist es beispielsweise möglich, a) nur CO zu entnehmen, b) nur Wasserstoff zu entnehmen, c) eine Synthesegasmischung aus CO und Wasserstoff zu entnehmen oder d) einen Strom von CO, einen Strom von Wasserstoff und einen Strom einer Synthesegasmischung (CO + Wasserstoff) zu entnehmen.

Weiterhin weist die Anlage 20 zur Erzeugung von Synthesegas der Fig. 2 einen ersten Wärmetauscher 25, einen zweiten Wärmetauscher 26 und dritten Wärmetauscher 27 auf. Der erste Wärmetauscher 25 steht mit der Verbindung 8 zwischen Kohlenwasserstoffkonverter 3 und CO₂-Konverter 9 in thermisch leitendem Kontakt und ist geeignet, der Verbindung ggf. überschüssige Wärme zu entziehen, die nicht für das Erreichen der Umwandlungstemperatur im CO₂-Konverter 9 erforderlich ist, oder Wärme aus anderen Bereichen der Anlage zuzuführen, sofern dies erforderlich ist.

Der zweite Wärmetauscher 26 steht mit der Verbindung zwischen dem CO₂-Konverter 9 und dem Mischer 21 in thermisch leitendem Kontakt und ist geeignet, der Verbindung und somit dem darin befindlichen heißen CO überschüssige Wärme zu entziehen. Diese kann zum Beispiel zum Vorheizen des in den CO₂-Konverter 9 eingeleiteten CO₂ verwendet werden. Hierfür würde sich insbesondere ein sogenannter Gegenstrom-Wärmetauscher anbieten, wie er in der Technik bekannt ist.

Der dritter Wärmetauscher 27 steht mit der Verbindung zwischen dem Kohlenwasserstoffkonverter 3 und dem Mischer 21 in thermisch leitendem Kontakt und ist geeignet, der Verbindung und somit dem darin befindlichen heißen Wasserstoff überschüssige Wärme zu entziehen. Die am ersten, zweiten oder dritten Wärmetauscher abgeführte Wärme kann zum Beheizen anderer Bereiche der Anlage insbesondere zum Warmhalten des CO₂-Konverters oder zum Vorwärmen des in den CO₂-Konverter eingeleiteten CO₂ verwendet werden. Ein Teil der Wärme kann auch beispielsweise über einen Dampferzeuger und eine Dampfturbine oder eine andere geeignete Vorrichtung in Strom umgewandelt werden.

Der Betrieb der Anlage 20 zur Erzeugung von Synthesegas gleicht, was dem Betrieb des Kohlenwasserstoffkonverters 3 und des CO₂-Konverters 9 betrifft, dem oben beschriebenen Betrieb der Anlage 1 gemäß Fig. 1. Bei der Anlage 20 zur Erzeugung von Synthesegas wird abhängig von der erwünschten Zusammensetzung des Synthesegases im Mischer 21 ein erwünschtes Mischungsverhältnis von Wasserstoff zu CO eingestellt und am Synthesegasausgang 24 des Mischers ausgeleitet, wobei der Wasserstoff bevorzugt, wie dargestellt, aber nicht zwingend über den Kohlenwasserstoffkonverter 3 zur Verfügung gestellt wird. Es sind hier auch andere Wasserstoffquellen, insbesondere auch ein zweiter Kohlenwasserstoffkonverter 3 denkbar, insbesondere ein Niedertemperatur-Konverter. Falls nicht die gesamte zur Verfügung stehende Menge an CO und/oder die gesamte zur Verfügung stehende Menge an H₂ verwendet werden, können die nicht im Mischer zusammengeführten Anteile der jeweiligen Gase CO oder H₂ jeweils einzeln weiterverarbeitet werden.

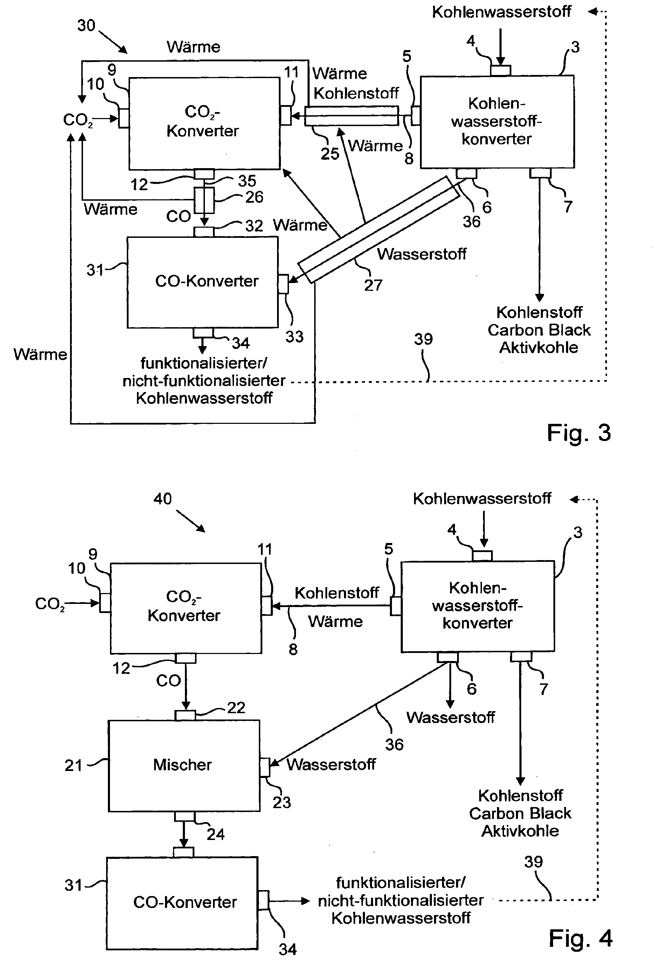

Fig. 3 zeigt eine Anlage 30 zur Erzeugung von synthetischen funktionalisierten und/oder nicht-funktionalisierten Kohlenwasserstoffen, welche eine Anlage 1 zur Umwandlung von Kohlendioxid in Kohlenmonoxid (wie in Fig. 1 gezeigt) und einen CO-Konverter 31 aufweist. Der Anlagenteil, der der Anlage 1 entspricht wird nicht näher erläutert, um Wiederholungen zu vermeiden. Der CO-Konverter 31 ist stromabwärts zum CO₂-Konverter 9 angeordnet und weist einen CO-Eingang 32 zum Einleiten von CO, einen H₂-Eingang 33 zum Einleiten von Wasserstoff und einen Kohlenwasserstoffausgang 34 zum Ausleiten von synthetischen funktionalisierten und/oder nicht-funktionalisierten Kohlenwasserstoffe auf. Der CO-Eingang 32 des CO-Konverters 31 ist mit dem CO-Ausgang 12 des CO₂-Konverters 9 durch eine CO-Verbindung 35 verbunden. Der H₂-Eingang 33 des CO-Konverters 31 ist durch eine H₂-Verbindung 36 mit dem H₂-Ausgang 6 des Kohlenwasserstoffkonverters 3 verbunden.

Die Anlage 30 zur Erzeugung von Kohlenwasserstoffen weist optional auch die in Verbindung mit der Anlage 20 (Fig. 2) beschriebenen Wärmetauscher 25, 26, 27 auf, die in der oben beschriebenen Weise arbeiten (siehe Beschreibung der Fig. 2).

Der CO-Konverter 31 kann ein beliebiger CO-Konverter zur Herstellung von synthetischen, funktionalisierten und/oder nicht-funktionalisierten Kohlenwasserstoffen sein. In der gezeigten Ausführungsform der Fig. 3, ist der CO-Konverter bevorzugt entweder ein Fischer-Tropsch-Konverter, ein Bergius-Pier-Konverter oder ein Pier-Konverter mit einem entsprechenden Katalysator und einer Temperatur und/oder Druck-Steuereinheit.

In einer Ausführungsform weist der CO-Konverter 31 einen Fischer-Tropsch-Konverter auf. Ein Fischer-Tropsch-Konverter wandelt katalytisch ein Synthesegas zu Kohlenwasserstoffen und Wasser um. Dem Fachmann sind verschiedene Ausführungen von Fischer-Tropsch-Reaktoren und Fischer-Tropsch-Verfahren bekannt, die hier nicht im Detail dargestellt werden sollen. Die Hauptreaktionsgleichungen lauten wie folgt:

n CO + (2n+1) H₂→ CₙH₂ₙ₊₂ + n H₂O für Alkane

n CO + (2n) H₂→ CₙH₂ₙ + n H₂O für Alkene

n CO + (2n) H₂→ CₙH₂ₙ₊₁OH + (n-1) H₂O für Alkohole

Die Fischer-Tropsch-Verfahren können als Hochtemperatur-Verfahren oder als Niedrigtemperatur-Verfahren durchgeführt werden, wobei die Prozesstemperaturen im Allgemeinen zwischen 200 und 400°C liegen. Bekannte Varianten des Fischer-Tropsch-Verfahrens sind u.a. die Hochlast-Synthese, die Synthol-Synthese und das SMDS-Verfahren der Firma Shell (SMDS = Shell Middle Distillate Synthesis). Durch einen Fischer-Tropsch-Konverter wird typischerweise eine Kohlenwasserstoffverbindung aus Flüssiggasen (Propan, Butan), Benzin, Kerosin (Dieselöl), Weichparaffin, Hartparaffin, Methanol, Methan- Dieselkraftstoff oder eine Mischung mehrerer derselben erzeugt. Die Fischer-Tropsch-Synthese ist exotherm, wie dem Fachmann bekannt ist. Die Reaktionswärme aus dem Fischer-Tropsch-Verfahren kann mittels eines (in den Figuren nicht gezeigten) Wärmetauschers, beispielsweise zum Vorwärmen von CO₂ verwendet werden. Es wird beispielsweise eine zweistufige Vorwärmung des in den CO₂-Konverter 9 eingeleiteten CO₂ in Betracht gezogen, wobei zuerst eine Vorwärmung mittels der Abwärme des CO-Konverters 31 (in der Ausführung als Fischer-Tropsch-Konverter) erfolgt und danach eine weitere Erwärmung des CO₂ mittels Wärme von einem oder mehreren der Wärmetauscher 25, 26, 27.

In einer alternativen Ausführungsform weist der CO-Konverter 31 einen Bergius-Pier-Konverter oder eine Kombination eines Pier-Konverters mit einem MtL-Konverter (MtL = Methanol-to-Liquid) auf.

In einem Bergius-Pier-Konverter läuft das dem Fachmann wohl bekannte Bergius-Pier-Verfahren ab, bei dem Kohlenwasserstoffe durch Hydrierung von Kohlenstoff mit Wasserstoff in einer exothermen chemischen Reaktion erzeugt werden. Das Spektrum der Ausgangsprodukte aus dem Bergius-Pier-Verfahren hängt von den Reaktionsbedingungen und der Reaktionsführung ab. Es werden hauptsächlich flüssige Endprodukte erhalten, die als Kraftstoffe verwendet werden können, beispielsweise Schwer- und Mittelöle. Bekannte Entwicklungen des Bergius-Pier-Verfahrens sind beispielsweise das Konsol-Verfahren und das H-Coal-Verfahren.

In der oben erwähnten Kombination eines Pier-Konverters mit einem MtL-Konverter wird zunächst Synthesegas nach dem bekannten Pier-Verfahren in Methanol umgewandelt. Der MtL-Konverter ist ein Konverter, in dem Methanol zu Benzin umgewandelt wird. Ein verbreitetes Verfahren ist das MtL-Verfahren der Fa. ExxonMobil bzw. Esso. Eingangsprodukt des MtL-Konverters ist typischerweise Methanol, beispielsweise aus dem Pier-Konverter. Das Ausgangsprodukt, das vom MtL-Konverter erzeugt wird, ist typischerweise Benzin, das zum Betreiben eines Ottomotors geeignet ist.

Zusammenfassend kann gesagt werden, dass in dem CO-Konverter 31, egal nach welchem der oben dargestellten Prinzipien dieser arbeitet, als Endprodukte funktionalisierte und/oder nicht-funktionalisierte Kohlenwasserstoffe synthetisch aus CO und H₂ hergestellt werden können. Die Prozesswärme, die bei der exothermen Umsetzung im CO-Konverter 31 auftritt, kann wiederum über einen Wärmetauscher zum Beheizen unterschiedlicher Bereiche der Anlage oder zum Erzeugen von Strom verwendet werden, um den Wirkungsgrad der hier beschriebenen Anlagen zu verbessern.

Sofern als Ausgangsprodukt des CO-Konverters 31 eine Mischung von Kohlenwasserstoffen vorliegt, die nach ihrer Auftrennung und Verfeinerung nicht direkt weiterverarbeitet oder als Fertigprodukt profitabel verkauft werden kann, können diese Kohlenwasserstoffe, beispielsweise Methan oder kurzkettige Paraffine in den hier beschriebenen Prozess zurückgeführt werden. Zu diesem Zweck weist die Anlage 30 eine Rückleitungsverbindung 39 auf, mit deren Hilfe ein Teil der synthetisch erzeugten Kohlenwasserstoffe zurück zum Kohlenwasserstoffeingang 4 des Kohlenwasserstoffkonverters 3 geleitet werden können. Je nach Zusammenstellung der zurückgeleiteten, synthetisch erzeugten Kohlenwasserstoffe erfolgt vor der Einleitung in den Kohlenwasserstoffeingang 4 noch eine Aufbereitung bzw. Abtrennung von nicht geeigneten Kohlenwasserstoffen.

Fig. 4 zeigt eine weitere Ausführungsform einer Anlage 40 zur Erzeugung von synthetischen, funktionalisierten und/oder nicht-funktionalisierten Kohlenwasserstoffen. Die Anlage 40 weist die oben beschriebene Anlage 20 zur Erzeugung eines Synthesegases sowie einen CO-Konverter 31 auf, wie er oben mit Bezug auf das Ausführungsbeispiel der Fig. 3 beschrieben wurde. Der Synthesegasausgang 24 des Mischers 21 ist mit dem CO-Konverter 31 verbunden. Der Mischer 21 ist dabei so eingestellt, dass er an seinem Synthesegasausgang 24 ein Synthesegas liefert, welches speziell für die Bedürfnisse des verwendeten CO-Konverters 31 angepasst ist. Die restlichen Elemente der Anlage 40 sind dieselben, wie oben beschrieben und auch der Betrieb der einzelnen Elemente erfolgt im Wesentlichen in der oben beschriebenen Art und Weise.

Es wird in Betracht gezogen, dass, je nach Größe der Anlage, eine Vielzahl von Kohlenwasserstoffkonvertern nebeneinander betrieben werden kann, um die erwünschte Umwandlungskapazität bereitzustellen. Wie oben erwähnt, können die Kohlenwasserstoffkonverter als Hochtemperatur-Kohlenwasserstoffkonverter und/oder als Niedertemperatur-Kohlenwasserstoffkonverter ausgeführt sein. Ein Hochtemperatur-Kohlenwasserstoffkonverter arbeitet bei Temperaturen von größer 1000°C und ein Niedertemperatur-Kohlenwasserstoffkonverter arbeitet bei Temperaturen zwischen 200 und 1000°C, wobei als zusätzliche Energiequelle zum Beispiel eine Mikrowelleneinheit vorgesehen ist, die Energie direkt in den Kohlenwasserstoff einkoppelt, um eine Aufspaltung in Kohlenstoff und Wasserstoff zu erreichen.

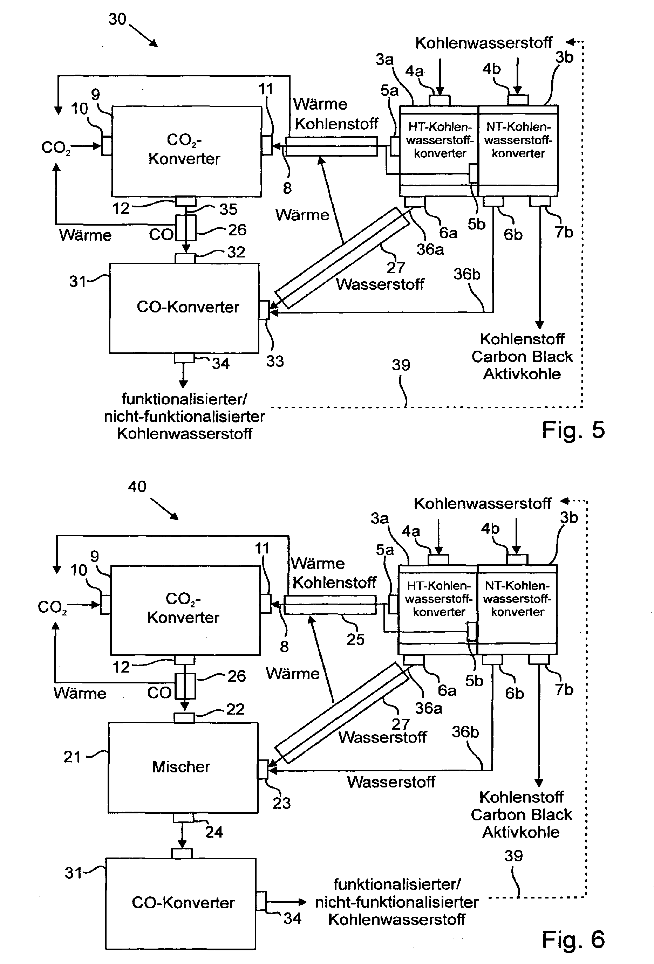

Als Beispiel einer Anlage mit einer Vielzahl von nebeneinander betriebenen Kohlenwasserstoffkonvertern zeigt Fig. 5 eine weitere Ausführung der Anlage 30 zur Erzeugung von synthetischen funktionalisierten und/oder nicht-funktionalisierten Kohlenwasserstoffen. In Fig. 5 werden dieselben Bezugszeichen, wie bei vorherigen Ausführungsformen verwendet, sofern gleiche oder ähnliche Elemente beschrieben werden. Bei der in Fig. 5 gezeigten Ausführung ist statt eines einzelnen Kohlenwasserstoffkonverters 3 eine Kombination eines Hochtemperatur-Kohlenwasserstoffkonverters 3a und eines Niedertemperatur-Kohlenwasserstoffkonverters 3b vorgesehen.

Der Hochtemperatur-Kohlenwasserstoffkonverter 3a weist einen Kohlenwasserstoffeingang 4a, einen ersten Ausgang 5a zum Ausleiten von Kohlenstoff und einen zweiten Ausgang 6a zum Ausleiten von Wasserstoff auf. Wiederum kann aber auch ein einzelner Ausgang 5a für eine Mischung (insbesondere ein Aerosol) aus Kohlenstoff und Wasserstoff vorgesehen sein. Der Ausgang 5a ist über eine Verbindung 8 mit dem Eingang 11 des CO₂-Konverters 9 verbunden. Der optionale Ausgang 6a des Hochtemperatur-Kohlenwasserstoffkonverters 3a ist mit dem H₂-Eingang 33 des CO-Konverters 31 verbunden. Der Hochtemperatur-Kohlenwasserstoffkonverter 3a kann optional einen in der Fig. 5 nicht gezeigten, weiteren Ausgang für Kohlenstoff aufweisen.

Der Niedertemperatur-Kohlenwasserstoffkonverter 3b weist einen Prozessraum mit einen Kohlenwasserstoffeingang 4b, einen ersten Ausgang 5b zum Ausleiten von Kohlenstoff, einen zweiten Ausgang 6b zum Ausleiten von Wasserstoff, und einen optionalen dritten Ausgang 7b zum Ausleiten von Kohlenstoff auf. Bevorzugt weist der Niedertemperatur-Kohlenwasserstoffkonverter 3b eine Trenneinheit auf, um Wasserstoff und Kohlenstoff nach Aufspaltung zu trennen und den jeweiligen Ausgängen zuzuleiten. Der erste Ausgang 5b ist optional über die Verbindung 8 mit dem Eingang 11 des CO₂-Konverters 9 verbunden, kann aber auch mit einer Kohlenstoff-Sammeleinheit verbunden sein. Der Ausgang 6b des Niedertemperatur-Kohlenwasserstoffkonverters 3b ist mit dem H₂-Eingang 33 des CO-Konverters 31 verbunden. Der optionale dritte Ausgang 7b ist mit einer Kohlenstoff-Sammeleinheit verbunden, aus der gesammelter Kohlenstoff zum Beispiel als Carbon Black, Aktivkohle oder einer anderen Form entnommen werden kann..

Der Kohlenwasserstoff, der in den Kohlenwasserstoffeingang 4a und in den Kohlenwasserstoffeingang 4b eingeleitet wird, kann der gleiche Kohlenwasserstoff sein oder es können unterschiedliche Kohlenwasserstoffe sein. In den Kohlenwasserstoffeingang 4a kann Kohlenwasserstoff aus einer ersten Kohlenwasserstoffquelle eingeleitet werden, beispielsweise Erdgas aus einem Erdgasvorrat. In den Kohlenwasserstoffeingang 4b des Niedertemperatur-Kohlenwasserstoffkonverters 3b kann hingegen zum Beispiel funktionalisierter und/oder nicht-funktionalisierter, synthetisch hergestellter Kohlenwasserstoff eingeleitet werden, beispielsweise über die zuvor erwähnte optionale Rückleitungsverbindung 39. Durch die Verwendung von mehreren, parallel betriebenen Kohlenwasserstoffkonvertern 3a, 3b ist die Anlage 30 leichter skalierbar, leichter steuerbar und es können unterschiedliche Arten von Kohlenstoff hergestellt werden.

Darüber hinaus kann zum Beispiel mit Vorteil der Hochtemperatur-Kohlenwasserstoffkonverter 3a verwendet werden, um "heißen" Kohlenstoff vorzugsweise mit einer Temperatur über 1000°C für die CO₂ Umwandlung im CO₂-Konverter 9 zu erzeugen. Hierbei kann der Hochtemperatur-Kohlenwasserstoffkonverter 3a insbesondere ohne eine Trenneinheit auskommen, da die durch die Aufspaltung gewonnene C-H₂ Mischung direkt in den CO₂-Konverter 9 eingeleitet werden kann. In diesem Fall gibt der CO₂-Konverter 9 dann am Ausgang zum Beispiel ein Synthesegas mit einem C-H₂ Mischungsverhältnis von ungefähr 1:1 aus.

Der Niedertemperatur-Kohlenwasserstoffkonverter 3b wird hingegen primär zur Erzeugung von zusätzlichem Wasserstoff verwendet, um für die Erzeugung eines Synthesegases oder einer C-H₂ Mischung mit einem C-H₂ Mischungsverhältnis von größer 1:1 insbesondere größer 1:2 für den CO-Konverter 31 bereitstellen zu können. Da hier kein Wärmetransfer vom Niedertemperatur-Kohlenwasserstoffkonverter 3b zu einem nachfolgenden Prozeß erforderlich ist, kann er Vorteilhaft mit Temperaturen unter 1000°C und bevorzugt bei der niedrigsten möglichen Temperatur betrieben werden.

Im Betrieb der Anlage 30 kann somit ein Teil des in den Kohlenwasserstoffkonvertern 3a, 3b erzeugten Kohlenstoffes (bevorzugt der aus dem Hochtemperatur-Kohlenwasserstoffkonverter 3a) in den CO₂-Konverter 9 eingeleitet werden, während ein anderer Teil (bevorzugt der aus dem Niedertemperatur-Kohlenwasserstoffkonverter 3b) als Grundstoff für die Erzeugung von weiteren Produkten aus dem Verfahren ausgeleitet werden kann. Solche Produkte sind beispielsweise Carbon Black bzw. Industrieruß, Aktivkohle, spezielle Modifikationen des Kohlenstoffs wie Carbon Discs und Carbon Cones usw., was als schwarzer, pulverförmiger Feststoff vorliegt. Dieser Kohlenstoff ist ein wichtiges technisches Produkt, welches beispielsweise als Füllstoff in der Gummiindustrie, als Pigmentruß für Druckfarben, Tuschen und Lacke oder als Ausgangsstoff zur Herstellung von elektrischen Bauteilen verwendet wird, beispielsweise für Zink-Kohle-Batterien und zur Herstellung von Kathoden oder Anoden. Gegebenenfalls überschüssiger Wasserstoff kann für die chemische Industrie ausgeleitet oder auch zur Erzeugung von Strom (durch Verbrennen) verwendet werden, wobei der Niedertemperatur-Kohlenwasserstoffkonverter 3b bevorzugt so betrieben wird, dass er nur den erforderlichen zusätzlichen Wasserstoff bereitstellt.

Fig. 6 zeigt eine alternative Ausführung der oben beschriebenen Anlage 40 zur Erzeugung von synthetischen, funktionalisierten und/oder nicht-funktionalisierten Kohlenwasserstoffen, bei der ebenfalls eine Vielzahl von parallel betriebenen Hochtemperatur- und/oder Niedertemperatur-Kohlenwasserstoffkonvertern vorgesehen ist.

Die in Fig. 6 gezeigte Anlage 40 zur Erzeugung von Kohlenwasserstoffen unterscheidet sich von der in Fig. 5 gezeigten Anlage 30 dadurch, dass vor dem CO-Konverter 31 ein Mischer 21 vorgeschaltet ist, der ein speziell für den CO-Konverter 31 zugeschnittenes Synthesegas zusammenmischt und an den CO-Konverter 31 liefert. Die in Fig. 6 dargestellten Elemente wurden oben bereits zuvor beschrieben und arbeiten auch nach den oben beschriebenen Prinzipien. Daher wird hier auf eine erneute detaillierte Beschreibung verzichtet, um Wiederholungen zu vermeiden.

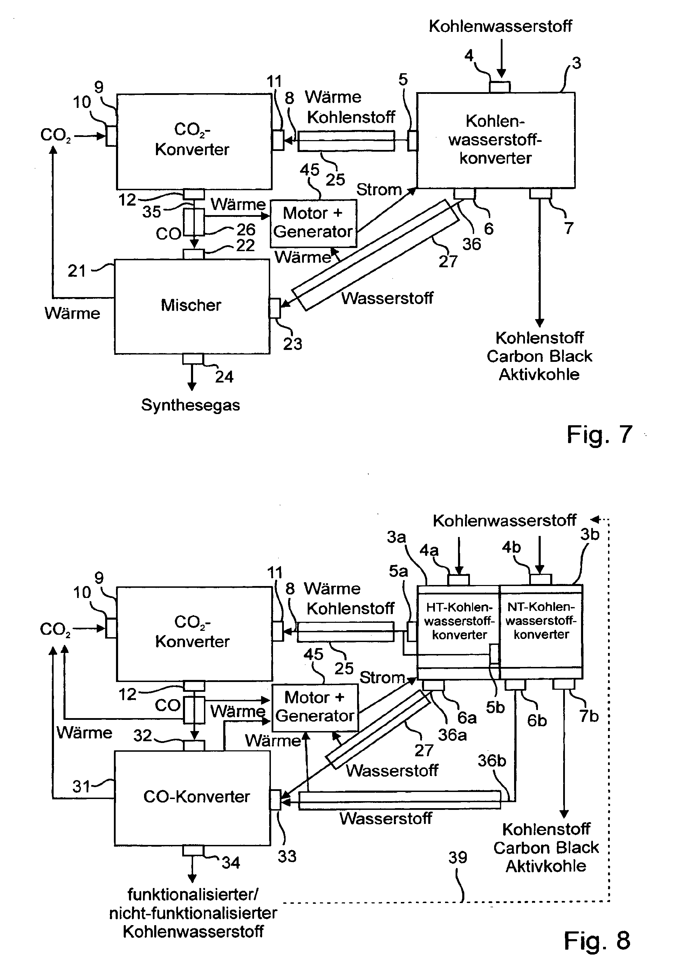

Die Fig. 7 und 8 zeigen Ausführungsformen der Anlagen 20 und 30, die einen ersten Wärmetauscher 25, einen zweiten Wärmetauscher 26 und einen dritten Wärmetauscher 27 aufweisen, welche jeweils mit einer Motor/Generator-Anordnung 45 verbunden sind. Die Motor/Generator-Anordnung 45 ist geeignet, aus überschüssiger Wärme von unterschiedlichen Stellen der Anlage zumindest teilweise elektrischen Strom zu erzeugen, welcher entweder in ein allgemeines Stromnetz eingespeist werden kann oder zum Betrieb der Anlage 20, 30 insbesondere des/der Kohlenwasserstoffkonverter verwendet werden kann. Weiter kann die Motor/Generator-Anordnung 45 mit einem in Fig. 8 nicht gezeigten Wärmetauscher am CO-Konverter 31 verbunden sein, der die Wärme ableitet, die bei dem exothermen Umwandlungsprozess entsteht, der im CO-Konverter 31 abläuft. So kann einerseits der CO-Konverter 31 in gesteuerter oder geregelter Weise gekühlt werden, was für die Prozessführung vorteilhaft ist, und andererseits elektrischer Strom erzeugt werden. Die Motor/Generator-Anordnung 45 kann irgendeine Vorrichtung sein, welche zur Verstromung von Wärmeenergie geeignet ist, beispielsweise eine Kombination aus einer Dampfturbine und einem Generator oder einem Kolbenmotor und einem Generator.

Die Motor/Generator-Anordnung 45 verstromt im Betrieb den Anteil der überschüssigen Wärme in der Anlage, d.h. die Wärme die nicht zum Erreichen der CO₂ Umwandlung verwendet wird.

Die Motor/Generator-Anordnung 45 und die Wärmetauscher 25, 26 und 27 sind optionale Elemente, die bei allen oben genannten Anlagen eingesetzt werden können. Auch der aus dem jeweiligen zweiten Ausgang 7, 7a, 7b ausgeleitete Kohlenstoff enthält aufgrund der Betriebstemperatur im jeweiligen Kohlenwasserstoffkonverter 3, 3a 3b eine beträchtliche Wärmeenergie. Abhängig von der erwünschten Temperatur des ausgeleiteten Kohlenstoffs kann ein Großteil dieser Wärmeenergie mittels in den Fig. nicht gezeigten Wärmetauschern abgeleitet werden und in den hier beschriebenen Verfahren weiterverwendet werden und/oder über die Motor/Generator-Anordnung 45 in Strom umgewandelt werden.

Bei den Anlagen 30 und 40 zur Erzeugung von synthetischen funktionalen und/oder nicht-funktionalen Kohlenwasserstoffen erfolgt die Abkühlung des Wasserstoffes aus dem Kohlenwasserstoffkonverter 3, 3a, 3b und/oder des CO aus dem CO₂-Konverter 9 nur soweit, dass die Betriebstemperatur des CO-Konverters 31 nicht unterschritten wird. Die Betriebstemperatur des CO-Konverters 31 liegt üblicherweise bei 200 bis 400°C, abhängig vom eingesetzten Verfahren.

Der Kohlenwasserstoffkonverter 3 kann bei allen oben beschriebenen Anlagen ein Hochtemperatur-Reaktor sein, welcher bei einer Temperatur von mehr als 1000°C arbeitet (z.B. ein Hochtemperatur-Kvaerner-Reaktor), oder ein Niedertemperatur-Reaktor, der bei einer Temperatur zwischen 200°C und 1000°C arbeitet (z.B. ein Niedertemperatur-Kvaerner-Reaktor). Ein derzeit erprobter Niedertemperatur-Reaktor arbeitet bei Temperaturen von 300 bis 800°C. Im Fall eines Niedertemperatur-Reaktor, der bei Temperaturen von 200 bis 800°C arbeitet, wird in Betracht gezogen, dass der eingeleiteten Kohlenstoff in der Verbindung 8 zwischen Kohlenwasserstoffkonverter 3 und CO₂-Konverter 9 vorgeheizt wird, da der CO₂-Konverter 9 bei Temperaturen von 800 bis 1000°C arbeitet. Aus den Fig. 7 und 8 wird weiter deutlich, dass eine Kombination von Hochtemperatur- und/oder Niedertemperatur-Kohlenwasserstoffkonvertern bei allen oben beschriebenen Anlagen 1, 20, 30 und 40 verwendet werden kann.

Ebenso kann bei allen oben beschriebenen Anlagen 1, 20, 30 und 40 ein Teil des im Kohlenwasserstoffkonverter 3, 3a, 3b erzeugten Kohlenstoffes als Carbon Black, Aktivkohle oder sonstiger Rohstoff entnommen werden, sofern dieser Kohlenstoff nicht im CO₂-Konverter 9 der Anlage 1, 20, 30, 40 umgesetzt wird. Weiterhin sei bemerkt, dass ebenfalls ein Teil des im Kohlenwasserstoffkonverter 3 erzeugten Wasserstoffes direkt aus dem Verfahren ausgeleitet und als Rohstoff vermarktet werden kann. Weiter kann optional bei allen oben beschriebenen Anlagen 30 und 40 eine Rückleitung von nicht erwünschten Anteilen der im CO-Konverter 31 synthetisch erzeugten funktionalisierten und/oder nicht-funktionalisierten Kohlenwasserstoffe zum Kohlenwasserstoffeingang 4, 4a, 4b des Kohlenwasserstoffkonverters 3 erfolgen.

Es wird in Betracht gezogen, dass das in den CO₂-Konverter 9 eingeleitete CO₂ ein Abgas aus einem Verbrennungskraftwerk darstellt, oder bei einem anderen industriellen Prozess anfällt. In letzter Zeit wird verstärkt darauf geachtet, dass wenig CO₂ in die Umwelt freigelassen wird, da CO₂ als Klimaschadstoff angesehen wird. In den genannten Abgasen ist das CO₂ mit anderen Gasen vermischt, unter anderem mit einem großen Anteil Stickstoff aus der Luft. Bei keiner der oben genannten Anlagen 1, 20, 30, 40 ist es nötig, den Stickstoff vor dem Einleiten in den CO₂-Konverter 9 abzutrennen. Sofern diese Gase nur in geringen Mengen vorliegen oder inert sind (z.B. Stickstoff), wird der Betrieb des CO₂-Konverters 9 durch diese mitgeführten Gase in der Regel nicht beeinträchtigt. Ein restlicher Sauerstoffanteil verbrennt im CO₂-Konverter 9 bei der hohen Betriebstemperatur und der Anwesenheit von Kohlenstoff.

Es folgen zur weiteren Veranschaulichung einige Anwendungsbeispiele:

### Beispiel 1: CO₂-freies Gaskraftwerk

Mithilfe eines Kvaerner-Reaktors als Kohlenwasserstoffkonverter 3 wird Methan in Kohlenstoff und Wasserstoff zerlegt. Dabei entstehen pro Atom Kohlenstoff zwei Moleküle Wasserstoff (CH₄→ C + 2 H₂). Ausgehend von einem konventionellen Gaskraftwerk mit hohem Wirkungsgrad, beispielsweise dem Typ Irsching IV der Firma Siemens AG mit 561 MW Nennleistung, wird das in der Abluft enthaltene CO₂ in den CO₂-Konverter 9 geleitet, etwa 1,5 Mio Tonnen im Jahr. Mit der Hälfte des Kohlenstoffs aus dem Kohlenwasserstoffkonverter 3 wird im CO₂-Konverter 9 das CO₂ aus der Abluft des Gaskraftwerks reduziert. Der Wasserstoff aus dem Kohlenwasserstoffkonverter 3 wird abgekühlt und die Abwärme mittels der Motor/Generator-Anordnung 45 verstromt. Das CO₂ aus dem Gaskraftwerk wird dann im CO₂-Konverter 9 über heißen Kohlenstoff geleitet und gemäß dem Boudouard-Gleichgewicht (CO₂ + C → 2 CO) in die doppelte Menge Kohlenmonoxid überführt. Das aus dem CO₂-Konverter 9 austretende heiße Kohlenmonoxid wird abgekühlt und die Abwärme verstromt. Das Kohlenmonoxid aus dem CO₂-Konverter 9 (Boudouard-Gleichgewicht) und der Wasserstoff aus dem Kohlenwasserstoffkonverter 3 (Kvaerner-Prozess) werden in einem CO-Konverter 31 (Fischer-Tropsch-Anlage) zu Kohlenwasserstoffen umgesetzt. Bevorzugt ist ein Heavy Paraffin Synthesis Modul mit anschließendem Heavy Paraffin Conversion Modul aus dem SMDS-Prozess (= Shell Middle Distillate Synthesis Prozess) der Firma Shell. Die Prozesswärme wird verstromt. Die erhaltenen Kohlenwasserstoffe hängen vom gewählten Fischer-Tropsch-Verfahren ab und können im SMDS-Verfahren der Firma Shell variiert werden.

Bei dem betrachteten Gaskraftwerk (561 MW) mit einem Wirkungsgrad von 60,4 %, einem Wirkungsgrad der Prozesswärmeverstromung von 60% und einem Wirkungsgrad der Abwärmeverstromung von 50% hat das Verfahren die folgenden Parameter:

| | | |
|---|---|---|
| Methanverbrauch | 2515 Mio N m³ CH₄/Jahr | |
| Elektrizitätserzeugung | 313 MW | |
| Carbon Black Produktion | 447000 t/Jahr | |
| Paraffinproduktion | 1,0 Mio t/Jahr | |
| CO₂-Emission | nahezu 0 | |
| Wirkungsgrad | Gaskraftwerk | 33,7 % |
| | Gesamt | 66,8 % |

### Beispiel 2: Gas-to-Liquid GtL-Anlage

Betreibt man die Anlage aus Beispiel 1 ohne Verstromung von Prozesswärme und Abwärme, so wird keine nennenswerte Menge Strom erzeugt. Es handelt sich dann um ein Verfahren zur Umwandlung gasförmiger Stoffe (Kohlendioxid und Methan) in flüssige Brennstoffe (Otto- und Dieselkraftstoffe, Kerosin), eine Gas-to-Liquid- bzw. GtL-Anlage. Im vorliegenden Beispiel wird zusätzlich Kohlenstoff produziert.

Die Parameter sind wie folgt:

| | |
|---|---|
| Methanverbrauch | 2 515 Mio N m³ CH₄/Jahr |
| Elektrizitätserzeugung | 0 MW |
| Carbon Black Produktion | 447 000 t/Jahr |
| Paraffinproduktion | 1,0 Mio t/Jahr |
| CO₂-Emission | nahezu 0 |

Die Erfindung wurde anhand spezieller Ausführungsformen und anhand einiger Beispiele näher erläutert, ohne hierauf beschränkt zu sein. Insbesondere sind die Elemente der einzelnen Ausführungsformen miteinander kombinierbar und/oder austauschbar, sofern kompatibel. Dem Fachmann werden sich jedoch zahlreiche Modifikationen und Abweichungen ergeben, die in den Umfang der nachfolgenden Ansprüche fallen. In einer besonders einfachen Ausgestaltung einer Anlage zur Erzeugung von synthetischen funktionalen und/oder nicht-funktionalen Kohlenwasserstoffen kann der CO₂-Konverter zum Beispiel als einfache Rohrleitung (zum Beispiel ein Ausgangsrohr eines keine Trenneinheit aufweisenden Hochtemperatur-Kohlenwasserstoffkonverters) ausgebildet sein in das eine CO₂-Leitung einmündet. Dabei sollte die CO₂-Leitung so in die Rohrleitung einmünden, dass eine gute Durchmischung der jeweiligen Medienströme erreicht wird. Die Rohrleitung sollte von einer Isolierung umgeben sein und könnte beispielsweise im Bereich eines Eingangsendes mit einer Heizeinheit in Verbindung stehen, um die Rohrleitung insbesondere zu Beginn des Betriebs auf eine Betriebstemperatur vorzuwärmen. Weiter stromabwärts könnte die Rohrleitung wiederum mit einem Wärmetauscher in Verbindung stehen, der überschüssige Wärme ableiten und gegebenenfalls zum Wärmen anderer Bereiche der Anlage und/oder zur Erzeugung von Strom einsetzen kann. In die Rohrleitung kann zusätzlich (zum Beispiel stromabwärts des Wärmetauschers) eine Zuleitung für Wasserstoff aufweisen, sodass dieselbe Rohrleitung nicht nur die Funktion eines CO₂-Konverters sondern auch eines Mischers zum Erzeugen eines Synthesegases übernehmen kann. Die Zuleitung für Wasserstoff kann zum Beispiel von einem Wasserstoffausgang eines Niedertemperatur-Kohlenwasserstoffkonverters (mit Trenneinheit) stammen. Ein Ausgangsende der Rohrleitung, an dem ein Synthesegas mit einem vorbestimmten Mischungsverhältnis ausgegeben werden kann, könnte dann in einem CO-Konverter enden.

## Patentansprüche

1. Verfahren zum Umwandeln von Kohlendioxid CO₂ in Kohlenmonoxid CO, welches folgende Schritte aufweist:
Aufspalten eines Kohlenwasserstoff enthaltenden Fluids zu Kohlenstoff und Wasserstoff in einem Kohlenwasserstoffkonverter durch einen Energieeintrag, der wenigstens teilweise durch Wärme geleistet wird,
wobei der Kohlenstoff und der Wasserstoff nach der Aufspaltung eine Temperatur von wenigstens 200 °C aufweist;
Leiten von wenigstens einem Teil des Kohlenstoffs, der aus der Aufspaltung gewonnen wurde, von dem Kohlenwasserstoffkonverter in einen CO₂-Konverter;
Einleiten von CO₂, das aus einem Verbrennungskraftwerk oder aus einem anderen Industrieprozess stammt, der geeignete Mengen an CO₂ erzeugt, in den CO₂-Konverter;
Vermischen des CO₂-Gases mit dem wenigstens einen Teil des Kohlenstoffes, der aus der Aufspaltung gewonnen wurde, wobei sich der durch die Aufspaltung gewonnene Kohlenstoff beim Vermischen mit dem CO₂-Gas um höchstens 50% in °C hinsichtlich seiner Temperatur nach der Aufspaltung abgekühlt hat;
Umwandeln wenigstens eines Teils des CO₂-Gases und des durch die Aufspaltung gewonnenen Kohlenstoffes in CO bei einer Temperatur von 800 bis 1700°C.

2. Verfahren zum Umwandeln von CO₂ in CO nach Anspruch 1, wobei die Aufspaltung bei einer Temperatur über 1000°C erfolgt und der Kohlenstoff mit einer Temperatur von wenigstens 800°C mit dem CO₂-Gas vermischt wird;
wobei die erforderliche Wärme zum Erreichen der Temperatur von 800 bis 1700°C für die CO₂ Umwandlung bevorzugt im Wesentlichen vollständig durch die Wärme, die für die Aufspaltung des Kohlenwasserstoff enthaltenden Fluids bereitgestellt wird, stammt.

3. Verfahren zum Umwandeln von CO₂ in CO nach einem der vorhergehenden Ansprüche, wobei der durch die Aufspaltung gewonnene Kohlenstoff und der durch die Aufspaltung gewonnene Wasserstoff gemeinsam mit dem CO₂-Gas vermischt werden.

4. Verfahren zum Umwandeln von CO₂ in CO nach einem der Ansprüche 1 oder 2, wobei der durch die Aufspaltung gewonnene Kohlenstoff vor dem Vermischen mit dem CO₂-Gas von dem durch die Aufspaltung gewonnenen Wasserstoff getrennt wird.

5. Verfahren zum Umwandeln von CO₂ in CO nach einem der vorhergehenden Ansprüche, wobei wenigstens ein Teil der Wärme wenigstens eines Teils des durch die Aufspaltung gewonnenen Kohlenstoffs und/oder Wasserstoffes und/oder des CO nach der Umwandlung zum Vorwärmen des CO₂-Gases vor dem Vermischen mit dem Kohlenstoff und/oder zur Erzeugung von elektrischem Strom verwendet wird, der insbesondere als Energieträger für den Energieeintrag für das Aufspalten des Kohlenwasserstoff enthaltenden Fluids bereitgestellt wird.

6. Verfahren zum Umwandeln von CO₂ in CO nach einem der vorhergehenden Ansprüche, wobei der Energieeintrag primär über ein Plasma insbesondere in einem Kvaerner-Reaktor erfolgt.

7. Verfahren zum Erzeugen eines Synthesegases, bei dem CO₂ in CO nach einem der vorhergehenden Ansprüche umgewandelt und mit Wasserstoff vermischt wird.

8. Verfahren zum Erzeugen eines Synthesegases nach Anspruch 7, wobei der Wasserstoff durch Aufspalten eines Kohlenwasserstoff enthaltenden Fluids zu Kohlenstoff und Wasserstoff durch einen Energieeintrag, der wenigstens teilweise durch Wärme geleistet wird, erzeugt wird; und
wobei insbesondere wenigstens ein Teil des Wasserstoffs durch Aufspalten eines Kohlenwasserstoff enthaltenden Fluids bei einer Temperatur unter 1000°C, insbesondere unter 600°C mittels eines Mikrowellenplasmas erzeugt wird.

9. Verfahren zum Erzeugen von synthetischen, funktionalisierten und/oder nicht-funktionalisierten Kohlenwasserstoffen, bei dem zunächst ein Synthesegas nach einem der Ansprüche 7 oder 8 erzeugt und dieses mit einem geeigneten Katalysator in Kontakt gebracht wird, um eine Umwandlung des Synthesegases in synthetische, funktionalisierte und/oder nicht-funktionalisierte Kohlenwasserstoffe zu bewirken, wobei die Temperatur des Katalysators und oder des Synthesegases auf einen vorbestimmten Temperaturbereich gesteuert oder geregelt wird.

10. Verfahren zum Erzeugen von synthetischen, funktionalisierten und/oder nicht-funktionalisierten Kohlenwasserstoffen nach Anspruch 9, wobei die Umwandlung des Synthesegases mittels eines der folgenden Verfahren erfolgt: Fischer-Tropsch-Verfahren, SMDS-Verfahren, Bergius-Pier-Verfahren, Pier-Verfahren oder eine Kombination aus einem Pier-Verfahren mit einem MtL-Verfahren.

11. Vorrichtung zum Umwandeln von Kohlendioxid CO₂ in Kohlenmonoxid CO, die folgendes aufweist:
einen Kohlenwasserstoffkonverter zum Aufspalten eines Kohlenwasserstoff enthaltenden Fluids in Kohlenstoff und Wasserstoff, der wenigstens einen Prozessraum mit wenigstens einem Eingang für ein Kohlenwasserstoff enthaltendes Fluid und wenigstens einem Ausgang für Kohlenstoff und/oder Wasserstoff und wenigstens eine Einheit zum Einbringen von Energie in den Prozessraum, die wenigstens teilweise aus Wärme besteht, aufweist;
einen CO₂-Konverter zur Umwandlung von CO₂ in CO, der wenigstens einen weiteren Prozessraum mit wenigstens einem Eingang für CO₂ zum Einleiten von CO₂ aus einem Verbrennungskraftwerk oder einem anderen Industrieprozess in den CO₂-Konverter, wenigstens einem Eingang für wenigstens Kohlenstoff und wenigstens einem Ausgang aufweist, wobei der Eingang für wenigstens Kohlenstoff direkt mit dem wenigstens einem Ausgang des Kohlenwasserstoffkonverters verbunden ist.

12. Vorrichtung zum Umwandeln von Kohlendioxid CO₂ in Kohlenmonoxid CO nach Anspruch 11, wobei die wenigstens eine Einheit zum Einbringen von Energie in den Prozessraum so ausgebildet ist, dass sie wenigstens lokal Temperaturen über 1000°C erzeugen kann, und/oder die wenigstens eine Einheit zum Einbringen von Energie in den Prozessraum bevorzugt eine Plasmaeinheit in einem Kvaerner-Reaktor, insbesondere eine Mikrowellen-Plasmaeinheit aufweist.

13. Vorrichtung zum Umwandeln von Kohlendioxid CO₂ in Kohlenmonoxid CO nach einem der Ansprüche 11 oder 12, wobei der Prozessraum des CO₂-Konverters durch ein Auslassrohr des Kohlenwasserstoffkonverters gebildet wird, das mit einer Einspeisung für CO₂-Gas verbunden ist, und/oder wobei ferner eine Trenneinheit zum Trennen des durch die Aufspaltung entstandenen Kohlenstoffs und des Wasserstoffs vorgesehen ist, und wobei getrennte Ausgänge für die getrennten Stoffe aus der Trenneinheit vorgesehen sind, wobei der Ausgang für Kohlenstoff mit dem CO₂-Konverter verbunden ist.

14. Vorrichtung zum Erzeugen eines Synthesegases, die eine Vorrichtung nach einem der Ansprüche 11 bis 13 aufweist, sowie wenigstens eine separate Zuleitung für Wasserstoff in den CO₂-Konverter oder einen stromabwärts befindlichen Mischraum.

15. Vorrichtung zum Erzeugen eines Synthesegases nach Anspruch 14, mit wenigstens einem weiteren Kohlenwasserstoffkonverter zum Aufspalten eines Kohlenwasserstoff enthaltenden Fluids in Kohlenstoff und Wasserstoff, der folgendes aufweist:
Wenigstens einen Prozessraum mit wenigstens einem Eingang für das Kohlenwasserstoff enthaltende Fluid,
wenigstens eine Einheit zum Einbringen von Energie in den Prozessraum, die wenigstens teilweise aus Wärme besteht,
eine Trenneinheit zum Trennen des durch die Aufspaltung entstandenen Kohlenstoffs und des Wasserstoffs mit getrennten Ausgängen für Kohlenstoff und Wasserstoff, wobei der Ausgang für Wasserstoff mit der separaten Zuleitung für Wasserstoff verbunden ist;
wobei der wenigstens eine weitere Kohlenwasserstoffkonverter vorzugsweise des Typs ist, der eine Aufspaltung bei Temperaturen unter 1000°C, insbesondere unter 600°C mittels eines Mikrowellenplasmas bewirkt.

16. Vorrichtung zum Umwandeln eines Synthesegases in synthetische, funktionalisierte und/oder nicht-funktionalisierte Kohlenwasserstoffe, die folgendes aufweist:
eine Vorrichtung nach einem der Ansprüche 14 oder 15; und
einen CO-Konverter mit einem Prozessraum in dem ein Katalysator angeordnet ist und Mittel zum Leiten des Synthesegases in Kontakt mit dem Katalysator, und eine Steuereinheit zum Steuern oder Regeln der Temperatur des Katalysators und/oder des Synthesegases auf eine vorbestimmte Temperatur.

17. Vorrichtung nach Anspruch 16, wobei der CO-Konverter einen der Folgenden aufweist: einen Fischer-Tropsch-Konverter, einen SMDS-Konverter, einen Bergius-Pier-Konverter, einen Pier-Konverter oder eine Kombination eines Pier-Konverters mit einen MtL-Konverter.

## Claims

1. A method for converting carbon dioxide CO₂ into carbon monoxide CO comprising the following steps:
decomposing a hydrocarbon containing fluid into carbon and hydrogen by means of introduction of energy in a hydrocarbon converter, the energy at least partially being provided by heat, wherein the carbon and the hydrogen have a temperature of at least 200°C after the decomposing step;
directing at least a portion of the carbon generated by the decomposing step from the hydrocarbon converter into a CO₂ converter;
introducing CO₂ gas from a power plant or from another industrial process into the CO₂ converter;
mixing the CO₂ gas with at least a portion of the carbon generated by the decomposing step, wherein upon mixing the carbon with the CO₂ gas, the carbon obtained by the decomposing step has cooled down by no more than 50% in °C with respect to its temperature after the decomposing step;
converting at least a portion of the CO₂ gas and the carbon obtained by the decomposing step into CO at a temperature between 800 and 1700°C.

2. The method for converting CO₂ into CO according to claim 1, wherein the decomposing step takes place at a temperature above 1000°C, and wherein the carbon is mixed with the CO₂ gas at a temperature of at least 800°C;
wherein the heat required to reach the temperature of between 800 and 1700°C for the CO₂ conversion originates essentially completely from the heat that is provided for decomposing the hydrocarbon containing fluid.

3. The method for converting CO₂ into CO according to one of the preceding claims,
wherein the carbon obtained by the decomposing step and the hydrogen obtained by the decomposing step are jointly mixed with the CO₂ gas.

4. The method for converting CO₂ into CO according to one of claims 1 or 2, wherein the carbon obtained by the decomposing step is separated from the hydrogen obtained by the decomposing step prior to the step of mixing the carbon with the CO₂ gas.

5. The method for converting CO₂ into CO according to one of the preceding claims,
wherein at least a portion of the heat of at least a portion of the carbon obtained by the decomposing step and/or of a portion of the hydrogen obtained by the decomposing step and/or of the CO after the conversion step is used to heat the CO₂ gas prior to mixing the CO₂ gas with carbon and/or is used for generating electricity, wherein the electricity may particularly be provided as energy carrier for introducing energy for decomposing the hydrocarbon containing fluid.

6. The method for converting CO₂ into CO according to one of the preceding claims,
wherein the energy is primarily introduced by means of a plasma, particularly in a Kvaerner reactor.

7. A method for generating a synthesis gas, wherein CO₂ is converted into CO according to one of the preceding claims; and wherein hydrogen is mixed with the CO.

8. The method for generating a synthesis gas according to claim 7, wherein the hydrogen is generated by decomposing a hydrocarbon containing fluid into carbon and hydrogen by introduction of energy that is at least partially provided by heat; and wherein particularly at least a portion of the hydrogen is generated by decomposing a hydrocarbon containing fluid at a temperature below 1000°C, particularly below 600°C, by means of a microwave plasma.

9. A method for generating synthetic functionalised and/or non-functionalised hydrocarbons, wherein at first a synthesis gas is generated according to the method of one of claims 7 or 8, and wherein the synthesis gas is brought into contact with a suitable catalyst in order to cause conversion of the synthesis gas into synthetic functionalised and/or non-functionalised hydrocarbons, wherein the temperature of the catalyst and/or the synthesis gas is open-loop controlled or close-loop regulated to a predetermined range of temperature.

10. The method for generating synthetic functionalised and/or non-functionalised hydrocarbons according to claim 9, wherein the conversion of the synthesis gas is carried out by means of one of the following: a Fischer-Tropsch process, a SMDS process, a Bergius-Pier process, a Pier process or a combination of a Pier process and a MtL process.

11. An apparatus for converting carbon dioxide CO₂ into carbon monoxide CO comprising:
a hydrocarbon converter for decomposing a hydrocarbon containing fluid into carbon and hydrogen, wherein the hydrocarbon converter comprises at least one process chamber having at least one inlet for a hydrocarbon containing fluid and at least one outlet for carbon and/or hydrogen and wherein the hydrocarbon converter comprises at least one unit for introducing energy into the process chamber, the energy consisting at least partially of heat;
a CO₂ converter for converting CO₂ into CO, the CO₂ converter comprising at least one further process chamber having at least one inlet for CO₂ adapted to introduce CO₂ from a power plant or from another industrial process into the CO₂ converter, at least one inlet for at least carbon, and at least one outlet, wherein the inlet for at least carbon is directly connected to the at least one outlet of the hydrocarbon converter.

12. The apparatus for converting carbon dioxide CO₂ into CO according to claim 11,
wherein the at least one unit for introducing energy into the process chamber is designed in such a way that it can generate, at least locally, temperatures above 1000°C and/or wherein the at least one unit for introducing energy into the process chamber comprises preferably a plasma unit in a Kvaerner reactor, particularly a microwave plasma unit.

13. The apparatus for converting carbon dioxide CO₂ into carbon monoxide CO according to one of claims 11 or 12, wherein the process chamber of the CO₂ converter is formed by an outlet pipe of the hydrocarbon converter, wherein the outlet pipe is connected to an inlet for CO₂ gas and/or wherein further a separation unit is provided for separating the carbon obtained by decomposing and the hydrogen obtained by decomposing, and wherein separate outlets are provided for the separated materials coming from the separation unit, wherein the outlet for carbon is connected to the CO₂ converter.

14. An apparatus for generating a synthesis gas comprising an apparatus according to one of claims 11 to 13 and at least one separate inlet pipe for hydrogen leading into the CO₂ converter or into a mixing chamber located downstream.

15. The apparatus for generating a synthesis gas according to claim 14 having at least one additional hydrocarbon converter for decomposing a hydrocarbon containing fluid into carbon and hydrogen, the hydrocarbon converter comprising:
at least one process chamber having at least one inlet for the hydrocarbon containing fluid;
at least one unit for introducing energy into the process chamber, the energy at least partially consisting of heat;
a separation unit for separating the carbon obtained by decomposing and the hydrogen obtained by decomposing, the separation unit having separate outlets for carbon and hydrogen, wherein the outlet for hydrogen is connected to the separate inlet for hydrogen;
wherein, preferably, the at least one additional hydrocarbon converter is of a type carrying out decomposing at temperatures below 1000°C, particularly below 600°C by means of a microwave plasma.

16. An apparatus for converting a synthesis gas into synthetic functionalised and/or non-functionalised hydrocarbons comprising:
an apparatus according to one of claims 14 or 15; and
a CO converter having a process chamber, in which a catalyst is located, and means for bringing the synthesis gas into contact with the catalyst, and a control unit open-loop controlling or close-loop regulating the temperature of the catalyst and/or the synthesis gas to a predetermined temperature.

17. The apparatus according to claim 16, wherein the CO converter comprises one of the following: a Fischer-Tropsch converter, a SMDS converter, a Bergius pier converter, a Pier converter or a combination of a Pier converter with a MtL converter.

## Revendications

1. Procédé de conversion de dioxyde de carbone CO₂ en monoxyde de carbone CO comprenant les étapes suivantes :
décomposer un fluide contenant un hydrocarbure en carbone et hydrogène par introduction d'énergie dans un convertisseur d'hydrocarbure, l'énergie étant au moins partiellement fournie par de la chaleur, le carbone et l'hydrogène ayant une température d'au moins 200°C après l'étape de décomposition ;
diriger au moins une partie du carbone produit par l'étape de décomposition à partir du convertisseur d'hydrocarbure dans un convertisseur de CO₂ ;
introduire du CO₂ gazeux à partir d'une installation de fourniture d'énergie ou à partir d'un autre processus industriel dans le convertisseur de CO₂ ;
mélanger le CO₂ gazeux avec au moins une partie du carbone produit par l'étape de décomposition, dans lequel, après mélange du carbone avec le CO₂ gazeux, le carbone obtenu par l'étape de décomposition s'était refroidi de pas plus de 50 % en °C par rapport à sa température après l'étape de décomposition ;
convertir au moins une partie du CO₂ gazeux et du carbone obtenu par l'étape de décomposition en CO à une température comprise entre 800 et 1700°C.

2. Procédé de conversion de CO₂ en CO selon la revendication 1, dans lequel l'étape de décomposition prend place à une température supérieure à 1000°C et dans lequel le carbone est mélangé au CO₂ gazeux à une température d'au moins 800° ;
dans lequel la chaleur requise pour atteindre une température comprise entre 800 et 1700°C pour la conversion de CO₂ provient pratiquement complètement de la chaleur qui est fournie par la décomposition du fluide contenant un hydrocarbure.

3. Procédé de conversion de CO₂ en CO selon l'une des revendications précédentes, dans lequel le carbone obtenu par l'étape de décomposition et l'hydrogène obtenu par l'étape de décomposition sont mélangés ensemble avec le CO2 gazeux.

4. Procédé de conversion de CO₂ en CO selon les revendications 1 ou 2, dans lequel le carbone obtenu par l'étape de décomposition est séparé de l'hydrogène obtenu par l'étape de décomposition avant l'étape de mélange du carbone avec le CO₂ gazeux.

5. Procédé de conversion de CO₂ en CO selon l'une des revendications précédentes, dans lequel au moins une partie de la chaleur d'au moins une partie du carbone obtenu par l'étape de décomposition et/ou une partie de l'hydrogène obtenu par l'étape de décomposition et/ou du CO après l'étape de conversion est utilisée pour chauffer le CO₂ gazeux avant mélange du CO₂ gazeux avec le carbone et/ou est utilisée pour produire de l'électricité, dans lequel l'électricité peut en particulier être fournie comme support d'énergie pour introduire de l'énergie pour décomposer le fluide contenant un hydrocarbure.

6. Procédé de conversion de CO₂ en CO selon l'une des revendications précédentes, dans lequel l'énergie est essentiellement introduite au moyen d'un plasma, en particulier dans un réacteur de Kvaerner.

7. Procédé de génération d'un gaz de synthèse, dans lequel du CO₂ est converti en CO selon l'une des revendications précédentes et dans lequel de l'hydrogène est mélangé au CO.

8. Procédé de génération d'un gaz de synthèse selon la revendication 7, dans lequel l'hydrogène est généré en décomposant un fluide contenant un hydrocarbure en carbone et en hydrogène par introduction d'énergie qui est au moins partiellement fournie par de la chaleur, et dans lequel en particulier au moins une partie de l'hydrogène est produit en décomposant un fluide contenant un hydrocarbure à une température inférieure à 1000°C, en particulier en dessous de 600°C, au moyen d'un plasma microonde.

9. Procédé de génération d'hydrocarbures de synthèse fonctionnalisés et/ou non fonctionnalisés, dans lequel au moins un gaz de synthèse est produit par le procédé selon l'une des revendications 7 ou 8, et dans lequel le gaz de synthèse est amené en contact avec un catalyseur adapté à provoquer la conversion du gaz de synthèse en hydrocarbures de synthèse fonctionnalisés et/ou non fonctionnalisés, dans lequel la température du catalyseur et/ou du gaz de synthèse est commandée en boucle ouverte ou régulée en boucle fermée dans une plage de températures prédéterminée.

10. Procédé de génération d'hydrocarbures de synthèse fonctionnalisés et/ou non fonctionnalisés selon la revendication 9, dans lequel la conversion du gaz de synthèse est effectuée par l'un des moyens suivants : processus Fischer-Tropsch, processus SMDS, processus Bergius-Pier, processus Pier ou combinaison d'un processus Pier et d'un processus MtL.

11. Dispositif de conversion de dioxyde de carbone CO₂ en monoxyde de carbone CO comprenant :
un convertisseur d'hydrocarbure pour décomposer un fluide contenant un hydrocarbure en carbone et en hydrogène, dans lequel le convertisseur d'hydrocarbure comprenant au moins une chambre de traitement ayant au moins une entrée pour un fluide contenant un hydrocarbure et au moins une sortie pour le carbone et/ou l'hydrogène, et dans lequel le convertisseur d'hydrocarbure comprend au moins un module pour introduire de l'énergie dans la chambre de traitement, l'énergie comprenant au moins partiellement de la chaleur ;
un convertisseur de CO₂ pour convertir le CO₂ en CO, le convertisseur de CO₂ comprenant au moins une autre chambre de traitement ayant au moins une entrée pour le CO₂ adaptée à introduire du CO₂ à partir d'une installation de fourniture d'énergie ou d'un autre processus industriel dans le convertisseur de CO₂, au moins une entrée pour au moins du carbone et au moins une sortie, dans lequel l'entrée pour au moins du carbone est directement connectée à ladite au moins une sortie du convertisseur d'hydrocarbure.

12. Dispositif de conversion de dioxyde de carbone CO₂ en CO selon la revendication 11, dans lequel ledit au moins un module pour introduire de l'énergie dans la chambre de traitement est conçu de façon à ce qu'il puisse produire, au moins localement, des températures supérieures à 1000°C, et/ou dans lequel ledit au moins un module pour introduire de l'énergie dans la chambre de traitement comprend de préférence un module de plasma dans un réacteur de Kvaerner, en particulier un module de plasma microonde.

13. Dispositif de conversion de dioxyde de carbone CO₂ en monoxyde de carbone CO selon l'une des revendications 11 ou 12, dans lequel la chambre de traitement du convertisseur de CO₂ est formée par un tube de sortie du convertisseur d'hydrocarbure, dans lequel le tube de sortie est connecté à une entrée pour le CO₂ gazeux et/ou dans lequel en outre un module de séparation est prévu pour séparer le carbone obtenu par la décomposition et l'hydrogène obtenu par la décomposition et dans lequel des sorties séparées sont prévues pour les matériaux séparés provenant du module de séparation, dans lequel la sortie pour le carbone est connectée au convertisseur de CO₂.

14. Dispositif de production d'un gaz de synthèse comprenant un dispositif selon l'une des revendications 11 à 13 et au moins un tube d'entrée séparé pour l'hydrogène conduisant au convertisseur de CO₂ et à une chambre de mélange disposée en aval.

15. Dispositif de production d'un gaz de synthèse selon la revendication 14, comprenant au moins un convertisseur d'hydrocarbure supplémentaire pour décomposer un fluide contenant un hydrocarbure en carbone et en hydrogène, le convertisseur d'hydrocarbure comprenant :
au moins une chambre de traitement ayant au moins une entrée pour le fluide contenant un hydrocarbure ;
au moins un module pour introduire de l'énergie dans la chambre de traitement, l'énergie consistant au moins partiellement en chaleur ;
un module de séparation pour séparer le carbone obtenu par décomposition et l'hydrogène obtenu par décomposition, le module de séparation ayant des sorties séparées pour le carbone et l'hydrogène, dans lequel la sortie d'hydrogène est connectée à l'entrée séparée d'hydrogène ;
dans lequel, de préférence, ledit au moins un convertisseur d'hydrocarbure supplémentaire est d'un type effectuant la décomposition à des températures inférieures à 1000°C, en particulier inférieures à 600°C au moyen d'un plasma microonde.

16. Dispositif de conversion d'un gaz de synthèse en des hydrocarbures de synthèse fonctionnalisés et/ou non fonctionnalisés, comprenant :
un dispositif selon l'une des revendications 14 ou 15 ; et
un convertisseur de CO comportant une chambre de traitement dans laquelle est disposée un catalyseur, et des moyens pour amener le gaz de synthèse en contact avec le catalyseur et un module de commande commandant en boucle ouverte ou régulant en boucle fermée la température du catalyseur et/ou du gaz de synthèse à une température prédéterminée.

17. Dispositif selon la revendication 16, dans lequel le convertisseur de CO comprend au moins l'un des moyens suivants : convertisseur Fischer-Tropsch, convertisseur SMDS, convertisseur Bergius-Pier, convertisseur Pier ou combinaison d'un convertisseur Pier et d'un convertisseur MtL.
